(19) Europäisches Patentamt
European Patent Office
Office européen des brevets

(11) EP 1 144 431 B1

(12) **EUROPÄISCHE PATENTSCHRIFT**

(45) Veröffentlichungstag und Bekanntmachung des Hinweises auf die Patenterteilung:
**21.12.2005 Patentblatt 2005/51**

(21) Anmeldenummer: **00907539.1**

(22) Anmeldetag: **09.02.2000**

(51) Int Cl.[7]: **C07J 53/00**, C07J 1/00, A61K 31/565, C07J 31/00, C07J 41/00, C07J 33/00, C07J 15/00

(86) Internationale Anmeldenummer:
**PCT/EP2000/001073**

(87) Internationale Veröffentlichungsnummer:
**WO 2000/047603 (17.08.2000 Gazette 2000/33)**

(54) **16-HYDROXYESTRATRIENE ALS SELEKTIV WIRKSAME ESTROGENE**

16-HYDROXYESTRATRIENES AS SELECTIVE ESTROGENS

16-HYDROXYESTRATRIENES SERVANT D'ESTROGENES AGISSANT DE MANIERE SELECTIVE

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **09.02.1999 DE 19906159**

(43) Veröffentlichungstag der Anmeldung:
**17.10.2001 Patentblatt 2001/42**

(60) Teilanmeldung:
**05075149.4 / 1 580 192**

(73) Patentinhaber: **Schering Aktiengesellschaft**
**13353 Berlin (DE)**

(72) Erfinder:
- **KÜNZER, Hermann**
  **D-13347 Berlin (DE)**
- **KNAUTHE, Rudolf**
  **D-13467 Berlin (DE)**
- **LESSL, Monika**
  **D-13467 Berlin (DE)**
- **FRITZEMEIER, Karl-Heinrich**
  **D-13505 Berlin (DE)**
- **HEGELE-HARTUNG, Christa**
  **D-45740 Mülheim a. d. Ruhr (DE)**
- **BÖMER, Ulf**
  **D-13347 Berlin (DE)**
- **MÜLLER, Gerd**
  **D-07745 Jena (DE)**
- **KOSEMUND, Dirk**
  **D-99091 Erfurt (DE)**

(56) Entgegenhaltungen:

| | |
|---|---|
| **EP-A- 0 367 576** | **WO-A-87/00175** |
| **WO-A-95/01988** | **WO-A-95/07925** |
| **WO-A-95/13076** | **WO-A-97/08188** |
| **WO-A-98/07740** | **WO-A-99/51243** |
| **CH-A- 537 916** | **CH-A- 538 460** |
| **CH-A- 557 805** | **DE-A- 2 007 417** |
| **DE-A- 2 007 464** | **DE-A- 2 209 244** |
| **DE-A- 2 653 558** | **DE-A- 4 239 946** |
| **FR-M- 4 339** | **FR-M- 5 099** |
| **GB-A- 804 789** | **GB-A- 823 955** |
| **US-A- 2 779 773** | **US-A- 3 079 408** |
| **US-A- 3 103 524** | **US-A- 3 166 473** |
| **US-A- 3 282 786** | **US-A- 3 449 383** |
| **US-A- 3 622 670** | **US-A- 3 766 224** |
| **US-A- 3 946 052** | **US-A- 4 212 864** |
| **US-A- 4 231 946** | **US-A- 4 592 869** |
| **US-A- 4 738 957** | **US-A- 5 587 496** |

- **G. STACK ET AL: "Relative Mitogenic Activities of Various Estrogens and Antiestrogens" STEROIDS., Bd. 54, Nr. 2, August 1989 (1989-08), Seiten 227-243, XP002137725 ELSEVIER SCIENCE PUBLISHERS, NEW YORK, NY., US ISSN: 0039-128X**
- **T. E. WIESE ET AL: "Induction of the Estrogen Specific Mitogenic Response of MCF-7 Cells by Selected Analogues of Estradiol-17.beta.: A 3D QSAR Study" JOURNAL OF MEDICINAL CHEMISTRY, Bd. 40, Nr. 22, 24. Oktober 1997 (1997-10-24), Seiten 3659-3669, XP002137726 WASHINGTON US**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- ANSTEAD G M ET AL: "The estradiol pharmacophore: Ligand structure-estrogen receptor binding affinity relationships and a model for the receptor binding site" STEROIDS: STRUCTURE, FUNCTION, AND REGULATION,US,ELSEVIER SCIENCE PUBLISHERS, NEW YORK, NY, Bd. 62, Nr. 3, 1. März 1997 (1997-03-01), Seiten 268-303, XP004057108 ISSN: 0039-128X
- TEDESCO R ET AL: "7alpha,11beta-disubstituted estrogens: probes for the shape of the ligand binding pocket in the estrogen receptor" BIOORGANIC & MEDICINAL CHEMISTRY LETTERS,GB,OXFORD, Bd. 7, Nr. 22, 18. November 1997 (1997-11-18), Seiten 2919-2924, XP004136557 ISSN: 0960-894X
- R. B. GABBARD ET AL: "STRUCTURE ACTIVITY RELATIONSHIPS OF ESTROGENS. EFECTS OF 14-DEHYDROGENATION AND AXIAL METHYL GROUPS AT C-7, C-9 AND C-11" STEROIDS., Bd. 41, Nr. 6, Juni 1983 (1983-06), Seiten 791-805, XP002137796 ELSEVIER SCIENCE PUBLISHERS, NEW YORK, NY., US ISSN: 0039-128X
- NAPOLITANO, ELIO ET AL: "11.beta.-Substituted Estradiol Derivatives, Potential High-Affinity Carbon-11-Labeled Probes for the Estrogen Receptor: A Structure-Affinity Relationship Study" J. MED. CHEM. (1995), 38(3), 429-34 , XP002149421
- E NAPOLITANO ET AL: "11.beta.-Substituted Estradiol Derivatives. 2. Potential Carbon-11-and Iodine-Labeled Probes for the Estrogen Receptor" JOURNAL OF MEDICINAL CHEMISTRY,US,AMERICAN CHEMICAL SOCIETY. WASHINGTON, Bd. 38, Nr. 14, 7. Juli 1995 (1995-07-07), Seiten 2774-2779, XP002100730 ISSN: 0022-2623
- CHEMICAL ABSTRACTS, vol. 94, no. 19, 11. Mai 1981 (1981-05-11) Columbus, Ohio, US; abstract no. 150860, JELINKOVA, MARTA ET AL: "A quantitative test for estrogenic activity using rat endometrium lactate dehydrogenase" XP002149426 & ACTA ENDOCRINOL. (COPENHAGEN) (1981), 96(3), 389-93 ,
- OJASOO, TIIU ET AL: "Unique steroid congeners for receptor studies" CANCER RES. (1978), 38(11, PT. 2), 4186-98 , XP000951497
- POIRIER D ET AL: "D-ring alkylamide derivatives of estradiol: effect on ER-binding affinity and antiestrogenic activity" BIOORGANIC & MEDICINAL CHEMISTRY LETTERS,GB,OXFORD, Bd. 6, Nr. 21, 5. November 1996 (1996-11-05), Seiten 2537-2542, XP004135909 ISSN: 0960-894X
- ARUNACHALAM T ET AL: "Iodoestrogens, syntheses, and interaction with uterine receptors" JOURNAL OF BIOLOGICAL CHEMISTRY,US,THE AMERICAN SOCIETY OF BIOLOGICAL CHEMISTS, INC.,, Bd. 254, Nr. 13, 10. Juli 1979 (1979-07-10), Seiten 5900-5905, XP002089838 ISSN: 0021-9258
- KANOJIA, RAMESH M. ET AL: "Synthesis and estrogenic properties of 17-epi-ethynylestradiol and its ether derivatives epimestranol and epiquinestrol" J. MED. CHEM. (1979), 22(12), 1538-41 , XP002149422
- PETERS R H ET AL: "17-DESOXY ESTROGEN ANALOGUES" JOURNAL OF MEDICINAL CHEMISTRY,US,AMERICAN CHEMICAL SOCIETY. WASHINGTON, Bd. 32, Nr. 7, Juli 1989 (1989-07), Seiten 1642-1652, XP002005625 ISSN: 0022-2623
- CHEMICAL ABSTRACTS, vol. 104, no. 7, 17. Februar 1986 (1986-02-17) Columbus, Ohio, US; abstract no. 51013, LI, JISONG ET AL: "Synthesis of 11-substituted estriols" XP002149427 -& "ELEVENTH COLLECTIVE INDEX, VOLUMES 96-105, 1982-1986, CHEMICAL SUBSTANCES Epoxynaphth-Ethanone" 1986 , CHEMICAL ABSTRACTS , COLUMBUS, OHIO, US XP002149423 & YAOXUE XUEBAO (1985), 20(3), 181-7 ,
- CHEMICAL ABSTRACTS, vol. 59, no. 7, 30. September 1963 (1963-09-30) Columbus, Ohio, US; abstract no. 7809c, D. L. COOK: "Effects of estrogenic steroids on blood lipids" XP002149428 & DRUGS AFFECTING LIPID METAB. PROC. SYMP. MILAN 1960, 1960, Seiten 204-211,
- CHEMICAL ABSTRACTS, vol. 102, no. 9, 4. März 1985 (1985-03-04) Columbus, Ohio, US; abstract no. 74834, TANG, ZHONGMING ET AL: "Structure and antiradiation activity relationship of estradiol derivatives" XP002149429 -& "ELEVENTH COLLECTIVE INDEX, VOLUMES 96-105, 1982-1986, CHEMICAL SUBSTANCES Epoxynaphth-Ethanone" 1986 , CHEMICAL ABSTRACTS , COLUMBUS, OHIO, US XP002149424 & YIYAO GONGYE (1984), (8), 10-14 ,
- CHEMICAL ABSTRACTS, vol. 96, no. 11, 15. März 1982 (1982-03-15) Columbus, Ohio, US; abstract no. 80149, POTTIER, J. ET AL: "Differences in the biotransformation of a 17.beta.-hydroxylated steroid, trenbolone acetate, in rat and cow" XP002149430 -& "ELEVENTH COLLECTIVE INDEX, VOLUMES 96-105, 1982-1986, CHEMICAL SUBSTANCES Epoxynaphth-Ethanone" 1986 , CHEMICAL ABSTRACTS , COLUMBUS, OHIO, US XP002149478 & XENOBIOTICA (1981), 11(7), 489-500 ,




(Forts. nächste Seite)

- **CHEMICAL ABSTRACTS, vol. 130, no. 26, 28. Juni 1999 (1999-06-28) Columbus, Ohio, US; abstract no. 350751, SHACKLETON, CEDRIC H. L. ET AL: "Equine type estrogens produced by a pregnant woman carrying a Smith-Lemli-Opitz syndrome fetus" XP002149431 & J. CLIN. ENDOCRINOL. METAB. (1999), 84(3), 1157-1159 ,**
- **CHEMICAL ABSTRACTS, vol. 101, no. 7, 13. August 1984 (1984-08-13) Columbus, Ohio, US; abstract no. 48830, KATZENELLENBOGEN, BENITA S.: "Biology and receptor interactions of estriol and estriol derivatives in vitro and in vivo" XP002149432 & J. STEROID BIOCHEM. (1984), 20(4B), 1033-7 ,**
- **CHEMICAL ABSTRACTS, vol. 119, no. 25, 20. Dezember 1993 (1993-12-20) Columbus, Ohio, US; abstract no. 271481, RZHEZNIKOV, VLADIMIR M.: "Method of preparing 9.alpha.,11.beta.-dihydroxy steroid 11-nitrates" XP002149433 -& "THIRTEENTH COLLECTIVE INDEX, VOLUMES 116-125, 1992-1996, CHEMICAL SUBSTANCES, DOC-Ethane, trib..." 1996 , CHEMICAL ABSTRACTS , COLUMBUS OHIO, US XP002149425 23 & SU 1 761 760 A (ALL-UNION ONCOLOGICAL RESEARCH CENTER, USSR) 15. September 1992 (1992-09-15)**
- **CHEMICAL ABSTRACTS, vol. 58, no. 5, 4. März 1963 (1963-03-04) Columbus, Ohio, US; abstract no. 4789a, ELAINE B. FELDMAN ET AL: "Antitumor, endocrine, and metabolic effects of 16.alpha.-estradiol dipropionate in women with breast cancer" XP002149434 & CANCER, Bd. 15, 1962, Seiten 1073-1075,**
- **CHEMICAL ABSTRACTS, vol. 85, no. 19, 8. November 1976 (1976-11-08) Columbus, Ohio, US; abstract no. 143353, FRASER, A. D. ET AL: "Preparation of tritiated substituted estratrienes" XP002149435 & J. LABELLED COMPD. RADIOPHARM. (1976), 12(2), 213-18 ,**


Bemerkungen:

Derzeit sind die WIPO-Publikationsdaten A3 nicht verfügbar.

## Beschreibung

### Feld der Erfindung

Die vorliegende Erfindung bezieht sich auf neue Verbindungen als pharmazeutische Wirkstoffe, die in vitro eine höhere Affinität an Estrogenrezeptorpräparationen von Rattenprostata als an Estrogenrezeptorpräparationen von Rattenuterus und in vivo vorzugsweise eine präferentielle Wirkung am Knochen im Vergleich zum Uterus und/oder ausgeprägte Wirkung hinsichtlich Stimulierung der Expression von 5HT2a-Rezeptor und - transporter aufweisen, deren Herstellung, ihre therapeutische Anwendung und pharmazeutischen Darreichungsformen, die die neuen Verbindungen enthalten.
Bei den chemischen Verbindungen handelt es sich um neuartige steroidale gewebeselektive Estrogene.

### Hintergrund der Erfindung

Etablierte Estrogentherapien zur Behandlung von hormondefizienzbedingten Beschwerden und die protektive Wirkung von Estrogenen auf Knochen, Gehirn, Gefäß und andere Organsysteme.
Die Effizienz von Estrogenen in der Behandlung von hormondefizienzbedingten Symptomen wie Hitzewallungen, Atrophie von Estrogenzielorganen und Inkontinenz, sowie die erfolgreiche Anwendung von Estrogen-Therapien zur Verhinderung von Knochenmasseverlust bei peri- und postmenopausalen Frauen, ist gut belegt und allgemein akzeptiert (Grady et al.1992, Ann Intern Med 117: 1016-1037). Ebenso ist gut dokumentiert, daß die Estrogenersatztherapie bei postmenopausalen Frauen oder bei Frauen mit anders bedingter ovarieller Dysfunktion, das Risiko von Herzkreislauferkrankungen gegenüber nicht estrogenbehandelten Frauen reduziert (Grady et al., loc. cit.).
Neuere Untersuchungen belegen zudem eine protektive Wirkung von Estrogenen gegen neurodegenerative Erkrankungen, wie z.B. Alzheimersche Krankheit (Henderson 1997, Neurology 48 (Suppl 7): S27-S35; Birge 1997, Neurology 48 (Suppl 7): S36-S41), eine schützende Wirkung auf Gehirnfunktionen, wie Gedächtnisleistung und Lernfähigkeit (McEwen et al. 1997, Neurology 48 (Suppl 7): S8-S15; Sherwin 1997, Neurology 48 (Suppl 7): S21-S26), sowie gegen hormondefiziensbedingte Stimmungsschwankungen (Halbreich 1997, Neurology 48 (Suppl 7): S16-S20).
Weiterhin hat sich Estrogenersatztherapie als effektiv hinsichtlich der Reduktion der Inzidenz von Kolorektalkarzinom erwiesen (Calle EF et al., 1995, J Natl Cancer Inst 87: 517-523).
In der herkömmlichen Estrogen- oder Hormonersatztherapie (Hormone Replacement Therapy = HRT) werden natürliche Estrogene, wie Estradiol und konjugierte Estrogene aus Pferdeurin entweder allein oder in Kombination mit einem Gestagen eingesetzt. Anstelle der natürlichen Estrogene können auch durch Veresterung erhaltene Derivate, wie z.B. das 17β-Estradiolvalerat, eingesetzt werden.
Wegen der stimulierenden Wirkung der verwendeten Estrogene auf das Endometrium, die zu einer Erhöhung des Endometriumkarzinomrisikos führt (Harlap S 1992, Am J Obstet Gynecol 166: 1986-1992), werden in der Hormonersatztherapie vorzugsweise Estrogen/Gestagen-Kombinationspräparate eingesetzt. Die gestagene Komponente in der Estrogen/Gestagen-Kombination vermeidet eine Hypertrophie des Endometriums, allerdings ist mit der gestagenhaltigen Kombination auch das Auftreten ungewünschter Zwischenblutungen verknüpft.
Eine neuere Alternative zu den Estrogen/Gestagen-Kombinationspräparaten stellen selektive Estrogene dar. Bisher werden unter selektiven Estrogenen solche Verbindungen verstanden, die estrogenartig auf Gehim, Knochen und Gefäßsystem, aufgrund ihrer antiuterotrophen (d.h. antiestrogenen) Partialwirkung aber nicht proliferativ auf das Endometrium wirken.
Eine Klasse von Substanzen, die das gewünschte Profil eines selektiven Estrogens teilweise erfüllen, sind die sogenannten, Selective Estrogen Receptor Modulators' (SERM) (R.F. Kauffman, H.U. Bryant 1995, DNAP 8 (9): 531-539). Es handelt sich hierbei um Partialagonisten des Estrogenrezeptorsubtyps, ERα'. Dieser Typ von Substanzen ist allerdings ineffektiv hinsichtlich der Therapie akuter postmenopausaler Beschwerden, wie z.B. Hitzewallungen. Als Beispiel für ein SERM sei das kürzlich für die Indikation Osteoporose eingeführte Raloxifen genannt.

### Estrogenrezeptor beta (ERβ)

Kürzlich wurde der Estrogenrezeptor-β (ERβ) als zweiter Subtyp des Estrogenrezeptors entdeckt (Kuiper et al. (1996), Proc. Natl. Acad. Sci. 93:5925-5930; Mosselman, Dijkema (1996) Febs Letters 392: 49-53; Tremblay et al. (1997), Molecular Endocrinology 11: 353-365). Das Expressionsmuster von ERβ unterscheidet sich von dem des ERα (Kuiper et al. (1996), Endocrinology 138: 863-870). So überwiegt ERβ gegenüber ERα in der Rattenprostata, während in Rattenuterus ERα gegenüber ERβ überwiegt. Im Gehirn wurden Areale identifiziert, in denen jeweils nur einer der beiden ER-Subtypen exprimiert wird (Shugrue et al. (1996), Steroids 61: 678-681; Li et al. (1997), Neuroendocrinology 66:63-67). ERβ wird u.a. in Arealen exprimiert, denen Bedeutung für kognitive Prozesse und 'Stimmung' zugewiesen wird (Shugrue et al. 1997, J Comparative Neurology 388:507-525).

Molekulare Targets für ERβ in diesen Gehirnarealen könnten der 5HT2a-Rezeptor und der Serotonintransporter sein (G. Fink & B.E.H. Sumner 1996 Nature 383:306; B. E.H. Sumner et al. 1999 Molecular Brain Research, in press). Der Neurotransmitter Serotonin (5-Hydroxytryptamin) ist an der Regulation einer Vielzahl von Prozessen beteiligt, die in der Menopause beeinträchtigt sein können. Insbesondere die Effekte der Menopause auf Stimmung und Kognition werden mit dem serotonergen System in Verbindung gebracht. Estrogenersatztherapie hat sich als effektiv hinsichtlich Behandlung dieser Estrogendefizienzbedingten Beschwerden erwiesen, möglicherweise durch Modulation von Serotoninrezeptor- und -Transporterexpression.

Weitere Organsysteme mit vergleichsweise hoher ERβ-Expression umfassen den Knochen (Onoe Y et al., 1997, Endocrinology 138:4509-4512), das Gefäßsystem (Register TC, Adams MR 1998, J Steroid Molec Biol 64: 187-191), den Urogenitaltrakt (Kuiper GJM et al. 1997, Endocrinology 138: 863-870), den Gastrointestinaltrakt (Campbell-Thopson 1997, BBRC 240: 478-483), sowie die Testis (Mosselmann S et al. 1996 Febs Lett 392 49-53) einschließlich der Spermatiden (Shugrue et al. 1998, Steroids 63: 498-504). Die Gewebeverteilung legt nahe, daß Estrogene über ERβ Organfunktionen regulieren. Daß ERβ in dieser Hinsicht funktionell ist, ergibt sich auch durch Untersuchungen an ERα- (ERKO) bzw. ERβ-(βERKO)-Knockout-Mäusen:Ovariektomie bewirkt Knochenmasseverlust in ERKO-Mäusen, der durch Estrogensubstitution aufgehoben werden kann (Kimbro et al. 1998, Abstract OR7-4, Endocrine Society Meeting New Orleans). Ebenso hemmt Estradiol in Blutgefäßen weiblicher ERKO-Mäuse die Gefäßmedia- und Glattmuskelzellproliferation (Iafrati MD et al. 1997, Nature Medicine 3: 545-548). Diese protektiven Wirkungen von Estradiol erfolgen in der ERKO-Maus vermutlich über ERβ.

Beobachtungen an ßERKO-Mäusen liefern einen Hinweis auf eine Funktion von ERβ in Prostata und Blase: bei älteren männlichen Mäusen treten Symptome von Prostata- und Blasenhyperplasie auf (Krege JH et al. 1998, Proc Natl Acad Sci 95: 15677-15682). Außerdem weisen weibliche (Lubahn DB et al. 1993, Proc Natl Acad Sci 90:11162-11166) und männliche ERKO-Mäuse (Hess RA et al. 1997, Nature 390: 509-512) sowie weibliche βERKO-Mäuse (Krege JH, 1998) Fertilitätsstörungen auf. Hierdurch wird die wichtige Funktion von Estrogenen hinsichtlich Aufrechterhaltung von Testis- und Ovarfunktion sowie Fertilität belegt.

Westerlind et al., 1998, beschreiben eine differentielle Wirkung von 16α-Hydroxyestron auf den Knochen einerseits und Reproduktionsorgane der weiblichen Ratte andererseits (Westerlind et al. 1998, J Bone and Mineral Res 13: 1023-1031).

Eigene Untersuchungen ergaben, daß 16α-Hydroxyestron 3-fach besser an den humanen Estrogenrezeptor β (ERβ), als an den humanen Estrogenrezeptor α (ERα) bindet. Der RBA-Wert der Substanz am Rattenprostataestrogenrezeptor ist 5-fach besser als der RBA-Wert der Substanz am Rattenuterusestrogenrezeptor. Die von Westerlind beschriebene Dissoziation der Substanz ist nach eigenen Erkenntnissen auf ihre Präferenz für ERβ im Vergleich zu ERα zurückzuführen.

Eine selektive Estrogenwirkung auf bestimmte Zielorgane könnte aufgrund der unterschiedlichen Gewebe- bzw. Organverteilung der beiden Subtypen des ERs durch subtypspezifische Liganden erreicht werden. Substanzen mit Präferenz für ERβ verglichen mit ERα im in vitro Rezeptorbindungstest wurden von Kuiper et al. beschrieben (Kuiper et al. (1996), Endocrinology 138: 863-870). Eine selektive Wirkung von subtypspezifischen Liganden des Estrogenrezeptors auf estrogensensitive Parameter in vivo wurde bisher nicht gezeigt.

Aufgabe der vorliegenden Erfindung ist es deshalb, Verbindungen bereitzustellen, die in vitro eine Dissoziation hinsichtlich Bindung an Estrogenrezeptorpräparationen von Rattenprostata und Rattenuterus und die in vivo vorzugsweise eine Dissoziation hinsichtlich Knochen- im Vergleich zur Uteruswirkung aufweisen. Die Verbindungen sollen in vitro eine höhere Affinität an Estrogenrezeptorpräparationen von Rattenprostata als an Estrogenrezeptorpräparationen von Rattenuterus und in vivo vorzugsweise eine höhere Potenz hinsichtlich Protektion gegen hormondefizienz-bedingten Knochenmasseverlust im Vergleich zur uterusstimulierenden Wirkung und/oder ausgeprägte Wirkung hinsichtlich Stimulierung der Expression von 5HT2a-Rezeptor und -transporter aufweisen.

Im weiteren Sinne soll durch die vorliegende Erfindung eine Struktur-Wirkungsbeziehung, zur Verfügung gestellt werden, die den Zugang zu Verbindungen gestattet, die das oben formulierte pharmakologische Profil, bessere estrogene Wirkung am Knochen als am Uterus, besitzen.

Erfindungsgemäß gelöst wird die vorstehende Aufgabe durch die Bereitstellung der 16α- und 16β-Hydroxyestra-1,3,5(10)-triene der allgemeinen Formel I

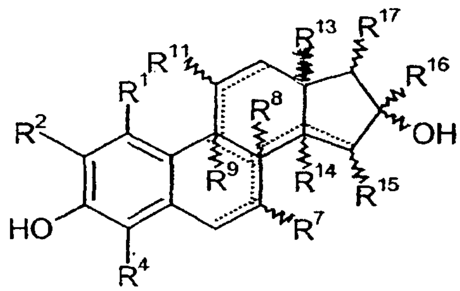

(I)

worin

| | |
|---|---|
| $R^7$ | ein α-oder β-ständiges Halogenatom, eine α-oder β-ständige, gerad- oder verzweigtkettige, gesättigte oder ungesättigte, gegebenenfalls teilweise oder vollständig fluorierte Alkylgruppe mit bis zu 10 Kohlenstoffatomen, eine gerad- oder verzweigtkettige, gesättigte oder ungesättigte Alkoxygruppe mit bis zu 6 Kohlenstoffatomen, ein gegebenenfalls substituierter Aryl- oder Heteroarylrest oder ein Wasserstoffatom; |
| $R^{13}$ | eine α- oder β-ständige Methyl- oder Ethyl- gruppe, |
| $R^1$, $R^2$, $R^4$, $R^8$, $R^9$, $R^{11}$, $R^{14}$, $R^{15}$, $R^{16}$ und $R^{17}$ | jeweils ein Wasserstoffatom bedeuten. |

Diese Verbindungen sind zur Herstellung eines Arzneimittels zur Behandlung und Prophylaxe estrogendefizienz-bedingter Krankheiten und Zustände geeignet.

Gemäß einer Variante der Erfindung werden vorzugsweise Verbindungen der allgemeinen Formel I verwendet,
worin

$R^7$ ein α-oder β-ständiges Fluoratom, eine α-oder β-ständige Methyl-, Ethyl-, Propyl- oder *i*-Propylgruppe, eine α oder β-ständige Trifluormethylgruppe oder ein Wasserstoffatom bedeuten.

16α-Hydroxy-17-Methylen-Estrogene wurden als antientzündlich wirksame und für die Therapie von immunologischen Erkrankungen, insbesondere Autoimmunerkrankungen, geeignete Verbindungen beschrieben (WO 97/08188).
Eine differenzierte Wirkung von 16α-Hydroxyestron wurde bereits von Westerling et al. beschrieben, s.o., nicht aber eine differenzierte Wirkung zwischen den Hirnfunktionen und dem Gefäßsystem einerseits und auf den Uterus andererseits.

3,16α-Dihydroxy-Estratrien wurde bereits von Stack und Gorski als "kurzzeitig wirkendes Estrogen" beschrieben (Stack, Gorski 1985).
Über eine Verwendung dieser zuletzt genannten Verbindung als selektives Estrogen ist bisher nichts bekannt.
3,16-Dihydroxy-estratriene, die in Position 16 zusätzlich mit einer Ethinylgruppe substituiert sind, wurden im Patent FR 5099 beschrieben. Die 16β-Ethinyl-verbindung kann als Mittel gegen erhöhte Cholesterinspiegel eingesetzt werden.

Die US-A-2,779,773 beschreibt Steroide aus der Gruppe der Estrogene und Androgene, in welchen die 16α-Hydroxygruppe verethert oder verestert ist. Sie bezieht sich außerdem auf eine Herstellungsmethode für diese Verbindugen. 3,16β-estradiol ist als Verbindung mit vernachlässigbarer estrogener Wirkung beschrieben, wohingegen das 3,16α-Epimer über beträchtliche estrogene Wirksamkeit verfügt.

GB-A-823,955 betrifft dieselben Steroide der Estrogen- und Androgengruppe wie in US-A-2,779,773.

Steroids, 54(2), 227-243 (1989) beschreibt und vergleicht die Fähigkeiten von verschiedenen Estrogenen und Antiestrogenen, die DNA-Synthese im präpuberalen Ratten-uterus zu stimulieren.

In den Verbindungen der allgemeinen Formeln I kann für ein Halogenatom immer ein Fluor-, Chlor-, Brom- oder Iodatom stehen; ein Fluoratom ist jeweils bevorzugt.

Die Alkoxygruppen in den Verbindungen der allgemeinen Formeln I und I' sowie in den nachstehend beschriebenen Teilstrukturen II und II' können jeweils 1 bis 6 Kohlenstoffatome enthalten, wobei Methoxy-, Ethoxy- Propoxy-

Isopropoxy- und t-Butyloxygruppen bevorzugt sind.

Beim einem Arylrest handelt es sich im Sinne der vorliegenden Erfindung um einen Phenyl-, 1- oder 2-Naphthylrest; der Phenylrest ist bevorzugt.

Wenn nicht ausdrücklich erwähnt, schließt Aryl immer auch einen Heteroarylrest mit ein. Beispiele für einen Heteroarylrest sind der 2-, 3- oder 4-Pyridinyl-, der 2- oder 3-Furyl-, der 2- oder 3-Thienyl-, der 2- oder 3-Pyrrolyl, der 2-, 4- oder 5-Imidazolyl-, der Pyrazinyl-, der 2-, 4- oder 5-Pyrimidinyl- oder 3- oder 4-Pyridazinylrest.

Als Substituenten für einen Aryl- oder Heteroarylrest seien zum Beispiel ein Methyl-, Ethyl-, Trifluormethyl-Pentafluorethyl-, Trifluormethylthio-, Methoxy-, Ethoxy-, Nitro-, Cyano-, Halogen- (Fluor, Chlor, Brom, Iod), Hydroxy-, Amino-, Mono($C_{1-8}$-alkyl)- oder Di($C_{1-8}$-alkyl)amino, wobei beide Alkylgruppen identisch oder verschieden sind, Di (aralkyl)amino, wobei beide Aralkylgruppen identisch oder verschieden sind, erwähnt.

Als Vertreter für gerad- oder verzweigtkettige Alkylgruppen mit 1-10 Kohlenstoffatomen sind beispielsweise Methyl, Ethyl, Propyl, Isopropyl, Butyl, Isobutyl, tert.-Butyl, Pentyl, Isopentyl, Neopentyl, Heptyl, Hexyl, Decyl zu nennen; Methyl, Ethyl, Propyl und Isopropyl sind bevorzugt.

Die Alkylgruppen können teilweise oder vollständig fluoriert oder substituiert sein durch 1-5 Halogenatome, Hydroxygruppen oder $C_1$-$C_4$-Alkoxygruppen.

Als perfluorierte Alkylgruppen seien beispielsweise Trifluormethyl, Pentafluorethyl und Nonafluorbutyl genannt. Vertreter der teilweise fluorierten Alkylgruppen sind zum Beispiel 2,2,2-Trifluorethyl, 5,5,5,4,4-Pentafluorpentyl, 9,9,9,8,8,7,7,6,6- Nonafluorhexyl etc..

Weitere Varianten der Erfindung sehen eine oder mehrere konjugierte Doppelbindungen in den Ringen B, C und D des Estratrien-Gerüsts vor:

Eine Doppelbindung zwischen den C-Atomen 6 und 7 oder zwischen den C-Atomen 7 und 8 oder zwischen den C-Atomen 8 und 9 oder zwischen den C-Atomen 9 und 11 oder zwischen den C-Atomen 8 und 14 oder zwischen den C-Atomen 14 und 15 oder Doppelbindungen zwischen den C-Atomen 6 und 7 sowie den C-Atomen 8 und 9 oder zwischen den C-Atomen 8 und 9 sowie den C-Atomen 14 und 15 oder zwischen den C-Atomen 6 und 7, den C-Atomen 8 und 9 sowie den C-Atomen 11 und 12 oder zwischen den C-Atomen 6 und 7, den C-Atomen 8 und 9 sowie den C-Atomen 14 und 15 oder zwischen den C-Atomen 6 und 7, den C-Atomen 8 und 9, den C-Atomen 11 und 12 sowie den C-Atomen 14 und 15.

Eine oder beide Hydroxylgruppen an den C-Atomen 3 und 16 können mit einer aliphatischen, gerad- oder verzweigtkettigen, gesättigten oder ungesättigten $C_1$-$C_{14}$-Mono- oder Polycarbonsäure oder einer aromatischen Carbonsäure oder mit einer α- oder β-Aminosäure verestert sein.

Als derartige Carbonsäuren zur Veresterung kommen beispielsweise in Betracht:

Monocarbonsäuren: Ameisensäure, Essigsäure, Propionsäure, Buttersäure, Isobuttersäure, Valeriansäure, Isovaleriansäure, Pivalinsäure, Laurinsäure, Myristinsäure, Acrylsäure, Propiolsäure, Methacrylsäure, Crotonsäure, Isocrotonsäure, Ölsäure, Elaidinsäure.

Dicarbonsäuren: Oxalsäure, Malonsäure, Bernsteinsäure, Glutarsäure, Adipinsäure, Pimelinsäure, Korksäure, Azelainsäure, Sebacinsäure, Maleinsäure, Fumarsäure, Muconsäure, Citraconsäure, Mesaconsäure.

Aromatische Carbonsäuren: Benzoesäure, Phthalsäure, Isophthalsäure, Terephthalsäure, Naphthoesäure, o-, m- und p-Toluylsäure, Hydratropasäure, Atropasäure, Zimtsäure, Nicotinsäure, Isonicotinsäure.

Als Aminosäuren kommen die dem Fachmann hinlänglich bekannten Vertreter dieser Substanzklasse in Frage, beispielsweise Alanin, β-Alanin, Arginin, Cystein, Cystin, Glycin, Histidin, Leucin, Isoleucin, Phenylalanin, Prolin etc..

Die 16-Oxy-funktion in den erfindungsgemäßen Verbindungen und in den nachstehend beschriebenen Strukturteilen kann sowohl in der α- als auch in der β-Position stehen.

Bevorzugt gemäß vorliegender Erfindung sind die nachstehenden Verbindungen

7α-Fluor-estra-1,3,5(10)-trien-3,16α-diol,
7α-Methyl-estra-1,3,5(10)-trien-3,16α-diol
7α-Fluor-estra-1,3,5(10)-trien-3,16β-diol,
7α-Methyl-estra-1,3,5(10)-trien-3,16β-diol
7α-Ethyl-estra-1,3,5(10)-trien-3,16α-diol
7α-Propyl-estra-1,3,5(10)-trien-3,16α-diol
7α-*i*-Propyl-estra-1,3,5(10)-then-3,16α-diol
7α-*i*-Propenyl-estra-1,3,5(10)-trien-3,16α-diol
7α-Phenyl-estra-1,3,5(10)-trien-3,16α-diol
7α-Methoxy-estra-1,3,5(10)-trien-3,16α-diol
7β-Ethyl-estra-1,3,5(10)-trien-3,16α-diol
7β-Propyl-estra-1,3,5(10)-trien-3,16α-diol

7β-*i*-Propyl-estra-1,3,5(10)-trien-3,16oc-diol
7β-*i*-Propenyl-estra-1,3,5(10)-trien-3,16α-diol
7β-Phenyl-estra-1,3,5(10)-trien-3,16α-diol
7β-Methoxy-estra-1,3,5(10)-trien-3,16α-diol
7α-Ethyl-estra-1,3,5(10)-trien-3,16β-diol
7α-Propyl-estra-1,3,5(10)-trien-3,16β-diol
7α-*i*-Propyl-estra-1,3,5(10)-trien-3,16β-diol
7α-*i*-Propenyl-estra-1,3,5(10)-trien-3,16β-diol
7α-Phenyl-estra-1,3,5(10)-trien-3,16β-diol
7α-Methoxy-estra-1,3,5(10)-trien-3,16β-diol
7β-Ethyl-estra-1,3,5(10)-trien-3,16β-diol
7β-Propyl-estra-1,3,5(10)-trien-3,16β-diol
7β-*i*-Propyl-estra-1,3,5(10)-trien-3,16β-diol
7β-*i*-Propenyl-estra-1,3,5(10)-trien-3,16β-diol
7β-Phenyl-estra-1,3,5(10)-trien-3,16β-diol
7β-Methoxy-estra-1,3,5(10)-trien-3,16β-diol
7α-Phenyl-8α-estra-1,3,5(10)-trien-3,16α-diol
18a-Homo-7α-phenyl-estra-1,3,5(10)-trien-3,16α-diol,
7α-Phenyl-8α-estra-1,3,5(10)-trien-3,16β-diol
18a-Homo-7α-phenyl-estra-1,3,5(10)-trien-3,16β-diol,
7α-Methyl-18a-homoestra-1,3,5(10)-trien-3,16α-diol
7α-Methyl-18a-homoestra-1,3,5(10)-trien-3,16β-diol
7α-Ethyl-18a-homoestra-1,3,5(10)-trien-3,16α-diol
7α-Ethyl-18a-homoestra-1,3,5(10)-trien-3,16β-diol

und von diesen wiederum insbesondere die Verbindungen

7α-Fluor-estra-1,3,5(10)-trien-3,16α-diol,
7α-Methyl-estra-1,3,5(10)-trien-3,16β-diol
7α-Methyl-estra-1,3,5(10)-trien-3,16α-diol
7α-Phenyl-estra-1,3,5(10)-trien-3,16β-diol
7β-Phenyl-estra-1,3,5(10)-trien-3,16β-diol
7β-Phenyl-estra- 1,3,5(10)-trien-3,16α-diol
7α-Ethyl-estra-1,3,5(10)-trien-3,16β-diol
7β-Ethyl-estra-1,3,5(10)-trien-3,16α-diol
7β-Ethyl-estra-1,3,5(10)-trien-3,16β-diol.

In der vorliegenden Patentanmeldung werden neuartige Strukturen für selektive Estrogene beschrieben, die in vitro Dissoziation hinsichtlich Bindung an Estrogenrezeptorpräparationen von Rattenprostata und Rattenuterus und die vorzugsweise in vivo eine Dissoziation hinsichtlich Knochen- im Vergleich zu Uteruswirkung aufweisen: über einen breiten Dosisbereich wirken die Substanzen knochenprotektiv ohne den Uterus zu stimulieren. Im gleichen Dosisbereich ist ihre Leberwirkung gering. Die Substanzen üben außerdem estrogenartige Wirkung auf das Gefäßsystem und Gehirnfunktionen aus.

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung dieser Verbindungen zur Herstellung von Arzneimitteln, insbesondere für die nachstehenden Indikationen.

Die Verbindungen können, sowohl nach oraler als auch parenteraler Gabe, für die folgenden Indikationen eingesetzt werden.

Die im vorliegenden Patent beschriebenen neuartigen selektiven Estrogene können als Einzelkomponente in pharmazeutischen Zubereitungen oder in Kombination insbesondere mit Antiestrogenen oder Gestagenen eingesetzt werden. Besonders bevorzugt ist die Kombination der selektiven Estrogene mit ERα-selektiven Antiestrogenen, oder mit Antiestrogenen, die peripherselektiv wirksam sind, d.h. die die Bluthirnschranke nicht passieren.

Die Substanzen und die sie enthaltenden Pharmaka sind besonders geeignet für die Behandlung peri- und postmenopausaler Beschwerden insbesondere Hitzewallungen, Schlafstörungen, Reizbarkeit, Stimmungsschwankungen, Inkontinenz, Vaginalatrophie, hormondefizienzbedingte Gemütserkrankungen. Ebenso sind die Substanzen für die Hormonsubstitution und die Therapie von hormondefizienz bedingten Beschwerden bei chirurgisch, medikamentös oder anders bedingter ovarieller Dysfunktion geeignet. Hierzu gehört auch die Vorbeugung gegen den Knochenmasseverlust bei postmenopausalen Frauen, bei hysterektomierten Frauen oder bei Frauen, die mit LHRH-Agonisten oder -Antagonisten behandelt wurden.

Die Verbindungen sind auch zur Linderung der Symptome der Andropause und Menopause, d.h. zur männlichen und weiblichen Hormonersatz-Therapie (HRT), und zwar sowohl zur Prävention als auch zur Behandlung, weiterhin zur Behandlung der mit einer Dysmenorrhoe einhergehenden Beschwerden sowie zur Behandlung der Akne geeignet.

Die Substanzen sind außerdem zur Prophylaxe gegen hormondefizienzbedingten Knochenmasseverlust und Osteoporose, zur Vorbeugung gegen Herzkreislauferkrankungen, insbesondere Gefäßerkrekungen wie Atherosklerose, zur Hemmung der Proliferation der arteriellen Glattmuskelzellen, zur Behandlung des primären pulmonaren Bluthochdrucks und zur Vorbeugung gegen hormondefizienzbedingte neurodegenerative Erkrankungen, wie Alzheimersche Krankheit, sowie hormondefizienzbedingte Beeinträchtigung von Gedächtnis- und Lernfähigkeit, einsetzbar.

Weiterhin sind die Substanzen zur Behandlung von entzündlichen Erkrankungen und Erkrankungen des Immunsystems, insbesondere Autoimmunerkrankungen, wie z.B. Rheumatoide Arthritis, einsetzbar.

Außerdem können die Verbindungen zur Behandlung männlicher Fertilitätsstörungen und prostatischer Erkrankungen Verwendung finden..

Die Verbindungen können auch in Kombination mit dem natürlichen Vitamin D3 oder mit Calcitriol-Analoga für den Knochenaufbau oder als unterstützende Therapie zu Therapien, welche einen Knochenmassenverlust verursachen (beispielsweise eine Therapie mit Glucocorticoiden, Chemotherapie) eingesetzt werden.

Schließlich können die Verbindungen der allgemeinen Formel I in Verbindung mit Progesteronrezeptor-Antagonisten verwendet werden, und zwar insbesondere zur Verwendung in der Hormonersatz-Therapie und zur Behandlung gynäkologischer Störungen.

Ein therapeutisches Produkt, enthaltend ein Estrogen und ein reines Antiestrogen für gleichzeitige, sequentielle oder getrennte Anwendung für die selektive Estrogentherapie perimenopausaler oder postmenopausaler Zustände ist bereits in der EP-A 0 346 014 beschrieben.

Die zu verabreichende Menge einer Verbindung der allgemeinen Formel I schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 µg/kg - 10 mg/kg Körpergewicht, vorzugsweise 0,04 µg/kg - 1 mg/kg Körpergewicht, je Tag betragen.
Beim Menschen entspricht dies einer Dosis von 0,8 µg bis 800 mg, vorzugsweise 3,2 µg bis 80 mg, täglich.

Eine Dosiseinheit enthält erfindungsgemäß 1,6 µg bis 200 mg einer oder mehrerer Verbindungen der allgemeinen Formel I.

Die erfindungsgemäßen Verbindungen und die Säureadditionssalze sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen oder deren Säureadditionssalze, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 ff., H. v. Czetsch-Lindenwald, Hilfsstoffe für Pharmazie und angrenzende Gebiete; Pharm. Ind., Heft 2, 1961, Seite 72 u. ff.: Dr. H. P. Fiedler, Lexikon der Hilfsstoffe für Pharmazie, Kosmetik und angrenzende Gebiete, Cantor KG. Aulendorf in Württemberg 1971.

Die Verbindungen können oral oder parenteral, beispielsweise intraperitoneal, intramuskulär, subkutan oder perkutan verabreicht werden. Die Verbindungen können auch in das Gewebe implantiert werden.
Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten.
Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.
Die Verbindungen lassen sich auch in Form einer Depotinjektion oder eines Implantatpräparats anwenden, die so formuliert sein können, daß eine verzögerte Wirkstoff-Freigabe ermöglicht wird.
Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Für die Herstellung von mit aktiven Verbindungen der allgemeinen Formel I beladenen Intravaginal- (z.B.

Vaginalringe) oder Intrauterinsystemen (z.B. Pessare, Spiralen, IUSs, Mirena®) für die lokale Verabreichung eignen sich verschiedene Polymere wie zum Beispiel Silikonpolymere, Ethylenvinylacetat, Polyethylen oder Polypropylen.

Um eine bessere Bioverfügbarkeit des Wirkstoffes zu erreichen, können die Verbindungen auch als Cyclodextrinclathrate formuliert werden. Hierzu werden die Verbindungen mit α-, β- oder γ-Cyclodextrin oder Derivaten von diesen umgesetzt (PCT/EP95/02656).

Erfindungsgemäß können die Verbindungen der allgemeinen Formel I auch mit Liposomen verkapselt werden.

**Methoden**

Estrogenrezeptorbindungsstudien

Die Bindungsaffinität der neuen selektiven Estrogene wurde in Kompetitionsexperimenten unter Verwendung von $^3$H-Estradiol als Ligand an Estrogenrezeptorpräparationen von Rattenprostata und Rattenuterus getestet. Die Präparation des Prostatacytosols und der Estrogenrezeptortest mit dem Prostatacytosol wurde, wie von Testas et al. (1981) beschrieben, durchgeführt (Testas J. et al., 1981, Endocrinology 109: 1287-1289).
Die Präparation von Rattenuteruscytosol, sowie der Rezeptortest mit dem ER-haltigen Cytosol wurden prinzipiell durchgeführt wie von Stack und Gorski, 1985, beschrieben (Stack, Gorski 1985, Endocrinology 117, 2024-2032) mit einigen Modifikationen wie bei Fuhrmann et al. (1995) beschrieben (Fuhrmann U. et al. 1995, Contraception 51: 45-52).

Die im vorliegenden Patent beschriebenen Substanzen weisen höhere Bindungsaffinität zu Estrogenrezeptor aus Rattenprostata als zu Estrogenrezeptor aus Rattenuterus auf. Dabei wird davon ausgegangen, daß ERβ gegenüber ERα in der Rattenprostata, in Rattenuterus ERα gegenüber ERβ überwiegt. Tabelle 1 zeigt, daß das Verhältnis der Bindung an Prostata- und Uterusrezeptor qualitativ mit dem Quotient der relativen Bindungsaffinität (RBA) an humanen ERβ und ERα von Ratte (nach Kuiper et al. (1996), Endocrinology 138: 863-870) übereinstimmt (Tabelle 1). Weiterhin wurde die Prädiktivität des 'Prostata-ER versus Uterus-ER-Testssystems' hinsichtlich gewebeselektiver Wirkung durch in vivo Untersuchungen bestätigt. Substanzen mit Präferenz für Prostata-ER sind in vivo hinsichtlich Knochen- und Uteruswirkung zugunsten der Wirkung am Knochen dissoziiert (Tabelle 2).

Knochenuntersuchungen

3 Monate alte weibliche Ratten werden ovarektomiert und unmittelbar nach der Operation 28 Tage lang 1mal täglich mit der Testverbindung behandelt. Die Applikation erfolgt subcutan in Arachisöl/Ethanol. Die Tiere werden am Tag nach der letzten Applikation getötet und Tibia sowie die Uteri entnommen. Die Uteri werden gewogen, fixiert und für histologische Untersuchungen aufgearbeitet. Die Bestimmung der Knochendichte erfolgt ex vivo an präparierten Langknochen mittels pQCT (Quantitative Computertomographie). Die Messungen werden im Abstand von 4 - 6 mm vom Gelenkkopf der proximalen Tibia durchgeführt.
Durch die Ovarektomie vermindert sich die Dichte des trabekulären Knochens im gemessenen Bereich von ca. 400 mg $Ca^{2+}/cm^3$ auf ca. 300 mg $Ca^{2+}/cm^3$. Durch die Behandlung mit einer Verbindung der allgemeinen Formel I gemäß vorliegender Erfindung wird der Abbau der Knochendichte verhindert bzw. gehemmt. Gemessen wurde die Knochendichte an der proximalen Tibia.

Tabelle 2 zeigt die Ergebnisse für Estra-1,3,5(10)-trien-3,16α-diol sowie für die Verbindungen der Beispiele 3, 4, 9, 10 und die Verbindungen 7β-Ethyl-estra-1,3,5(10)-trien-3,16β-diol, 7α-Ethyl-estra-1,3,5(10)-trien-3,16β-diol, 7β-Phenyl-estra-1,3,5(10)-trien-3,16β-diol, 7α-Phenyl-estra-1,3,5(10)-trien-3,16β-diol, 7β-Phenyl-estra-1,3,5(10)-trien-3,16α-diol und 7β-Ethyl-estra-1,3,5(10)-trien-3,16α-diol.
Sie zeigen eine höhere Bindungsaffinität am Estrogenrezeptor aus Rattenprostata als am Estrogenrezeptor aus Rattenuterus; für die Verbindung Estra-1,3,5(10)-trien-3,16α-diol ER(RBA) Rattenprostata = 50 und ER(RBA) Rattenuterus = 9. In vivo spiegelt sich dies in den stark unterschiedlichen Mengen Estra-1,3,5(10)-trien-3,16α-diol wider, die eine 50%ige Knochenprotektion [3μg/Tier] bzw. eine 50%ige Uterusstimulation [30μg/Tier] bewirken, bezogen auf den Knochenmasseverlust, der in ovarektomierten, unbehandelten weiblichen Ratten 28 Tage nach der Ovarektomie im Unterschied zu sham-oprierten, intakten Tieren meßbar ist.

Die Gefäßwirkung der erfindungsgemäßen Estrogene wird im Modell der ApoE-Knockout-Maus, wie von R. Elhage et al., 1997, beschrieben, ermittelt (Elhage R. et al. 1997, Arteriosclerosis, Thrombosis, and Vascular Biology 17: 2679-2684).

Zum Nachweis der Wirkung von Estrogenen auf die Gehirnfunktion wird die Oxytocinrezeptor mRNA-Expression als Surrogatparameter verwendet (Hrabovszky E et al. 1998, Endocrinology 1339: 2600-2604). Ovariektomierte Ratten werden über 7 Tage mit der Testsubstanz oder Vehikel behandelt (Applikation: subkutan oder oral, 6-mal täglich). Am Tag 7 nach der ersten Applikation werden die Tiere dekapitiert, das Uterusgewicht wird bestimmt und der Oxytocinrezeptor mRNA Spiegel wird mittels in situ Hybridisierung an geeigneten Gehimschnitten untersucht. Es werden die $ED_{50}$-Werte hinsichtlich Stimulierung von Utersuswachstum und Induktion der Oxytocinrezeptor mRNA bestimmt.

Eine andere Möglichkeit, die dissoziierte Estrogenwirkung der erfindungsgemäßen Substanzen in vivo nachzuweisen, besteht darin, nach Einmalapplikation der Substanzen bei Ratten Effekte auf die Expression von 5HT2a-Rezeptor- und Serotonintransporter-Protein- und mRNA-Level in ERβ-reichen Gehirnarealen zuvermessen. Vergleichend zum Effekt auf Serotoninrezeptor- und Transporterexpression wird der Effekt auf die LH-Sekretion gemessen. Substanzen mit höherer Bindung an den Rattenprosta- verglichen mit dem Rattenuterusestrogenrezeptor sind potenter hinsichtlich Erhöhung der Expression von Serotoninrezeptor- und transporter, im Vergleich zu ihrem positiven Effekt auf die LH-Ausschüttung. Die Dichte von Serotoninrezeptor und -Transporter wird an Gehirnschnitten mittels radioaktiver Liganden, die entsprechende mRNA mittels in situ Hybridisierung bestimmt. Die Methode ist in der Literatur beschrieben: G. Fink & B.E.H. Sumner 1996 Nature 383:306; B. E.H. Sumner et al. 1999 Molecular Brain Research.

**Herstellung der erfindungsgemäBen Verbindungen**

Für die Herstellung der erfindungsgemäßen Verbindungen, d.h. modifizierten/substituierten Derivaten der Estra-1,3,5(10)-trien-3,16ξ-diole, finden vor allem zwei generell anwendbare Synthesestrategien Verwendung.

Einerseits lassen sich insbesondere 3,16-geschützte Abkömmlinge der Estra-1,3,5(10)-trien-3,16ξ-diole, gegebenenfalls aber auch die freien Diole, für Modifikationen an einzelnen Positionen des Gerüstes einsetzen.

Andererseits bieten entsprechend modifizierte Estronanaloga, die in großer Zahl auf bekannten Wegen (charakteristische aber nicht einschränkende Syntheseverfahren, die zur Schaffung repräsentativer Substitutionsmuster am Estrongerüst, finden sich etwa in: C(7) Steroids 54, 1989, 71; C(8α) Tetrahedron Letters 1991, 743; C(8β) Tetrahedron Letters 1964, 1763; Tetrahedron 1969, 25, 4011; J. Org. Chem. 1970, 35, 468.

Bei der Einführung eines Substituenten in der Position 7 des Steroidgerüsts entstehen meist Gemische der 7α- und 7β-Stellungsisomeren. Diese lassen sich nach dem Fachmann geläufigen Methoden, beispielsweise chromatographisch, in die einzelnen Isomere auftrennen.

Für den Fall des C(3)-Methylethers von 8α-Estra-1,3,5(10)-trien-17-on (Bull. Sec. Chim. Fr. 1967, 561) wird eine ausführliche exemplarische Beschreibung angegeben. Nach Überführung des Ketons in ein Sulfonylhydrazon, im einfachsten Falle durch Umsetzung mit Phenylsulfonylhydrazid, erfolgt in einer Abbaureaktion die Bildung des C(16)-C(17) Olefins (Z. Chem. 1970, 10, 221-2; Liebigs Ann. Chem. 1981, 1973-81), an das in regio/stereokontrollierter Weise Hydrobromid angelagert wird. Reduktive Dehalogenierung und Entfernung der Schutzgruppe an C(3) ergeben den 16β-Alkohol, der nach bekannten Methoden in das 16α-Epimer überführt werden kann.

Eine weitere Variante für die Einführung der Hydroxylgruppe an C-Atom 16 besteht in der Hydroborierung der 16(17)-Doppelbindung mit sterisch anspruchsvollen Boranen. Von dieser Reaktion ist bekannt, daß sie zu 16-oxygenierten Produkten führt (Indian J. Chem. 1971, 9, 287-8). Dementsprechend ergibt die Umsetzung von 3-Methoxy-estra-1,3,5(10),16-tetraen und 3-Methoxy-18a-homoestra-1,3,5(10),16-tetraen mit 9-Borabicyclo[3.3.1]nonan nach der Oxidation mit alkalischem Wasserstoffperoxid 16α-Hydroxyestratriene. In untergeordnetem Maße werden bei dieser Reaktion die epimeren 16β-Hydroxysteroide gebildet. Nach der Spaltung der 3-Methoxygruppe werden Estra-1,3,5(10)-trien-3,16α-diole erhalten. Durch Inversion der Konfiguration an C-Atom 16, z. B. durch Mitsunobu-Reaktion (Synthesis 1980, 1), werden wiederum die 16β-Hydroxyestratriene erhalten.

Die breite Anwendbarkeit der soeben skizzierten Synthesewege wird an weiteren Beispielen, so etwa für 3-Methoxy-7α-methylestra-1,3,5(10)-trien-17-on (Helv. Chim. Acta 1967, 281) oder 1,3-Dimethoxyestra-1,3,5(10)-trien-17-on (J. Org. Chem. 1967, 32, 4078) demonstriert.

Die Herstellung der zentralen C(16)-C(17) olefinischen Zwischenstufen ist nicht auf die Arylsulfonylhydrazon-Methode beschränkt. Für den Fall, daß Substituenten am Steroidgerüst mit den basischen Reaktionsbedingungen der Olefinierung nicht verträglich sind, kommen andere Verfahren, insbesondere die Überführung der C(17) Ketone in Vinyljodide (Tetrahedron 1988,147) oder Enoltriflate (Tetrahedron Letters 1984,4821) und deren nachfolgende Reduktion als Alternative infrage.

Wählt man einen Syntheseweg über C(16)-Keto-Derivate, die anschließend in die C(15)b-bzw. durch Inversion in die C(16)a-Alkohole überführt werden, so stehen auch die Möglichkeiten zur C(17) → C(16)-Ketotransposition zur Auswahl. Für ein konkretes Beispiel sei auf J. Chem. Soc. Perk. 1, 1976, 1350 verwiesen.

Die erfindungsgemäßen Verbindungen der allgemeinen Formel I werden wie in den Beispielen beschrieben hergestellt. Durch analoge Vorgehensweise unter Verwendung homologer Reagenzien zu den in den Beispielen beschriebenen Reagenzien lassen sich weitere Verbindungen der allgemeinen Formel I erhalten.

Veretherung und/oder Veresterung freier Hydroxygruppen erfolgt nach dem Fachmann gängigen Methoden.

Die mit ϕ gekennzeichneten Beispiele gehören nicht zum Umfang der allgemeinen Formel I :

**Beispiel 1**

**8α-Estra-1,3,5(10)-trien-3,16β-diol**

**3-Methoxy-8α-estra-1,3,5(10)-trien-17-on-17-phenylsulfonylhydrazon**

Eine Suspension von 5,68 g (20 mmol) 3-Methoxy-8α-estra-1,3,5(10)-trien-17-on und 4,30 g (25 mmol) Benzolsulfonsäurehydrazid in 70 ml Ethanol versetzt man mit 3 Tropfen konzentrierter Salzsäure und läßt anschließend bei 80-90 °C Badtemperatur drei Stunden unter kräftigem Rühren reagieren. Nach Abkühlen der Reaktionslösung saugt man das ausgefallene Produkt ab, wäscht mit wenig kaltem Ethanol nach und trocknet das Hydrazon im Vakuum. Man erhält 8,10 g (92 %) Produkt, das bei 183-185 °C schmilzt.

**3-Methoxy-8α-estra-1,3,5(10),16-tetraen**

Eine Suspension von 8,10 g (18,5 mmol) des oben beschriebenen Hydrazons in 140 ml trockenem Ether kühlt man im Eisbad unter Feuchtigkeitsausschluß (Argonatmosphäre) auf 0 °C ab und versetzt tropfenweise mit 36 ml Methyllithium (57 mmol) in Ether. Nach erfolgter Zugabe entfernt man das Kältebad und rührt noch 3 Stunden bei Raumtemperatur. Zur Aufarbeitung kühlt man das Reaktionsgemisch auf 0 °C und versetzt unter kräftigem Rühren vorsichtig mit gesättigter wässriger Ammoniumchlorid-Lösung (30 ml). Diese Mischung wird mit Essigester versetzt, die organische Phase mit Wasser/Sole gewaschen und über Natriumsulfat getrocknet. Das Rohprodukt wird an Kieselgel chromatografiert (Hexan/Essigester, 95:5). Man erhält 3,60 g (72 %) Produkt.

**17α-Brom-3-methoxy-8α-estra-1,3,5(10)-trien-16β-ol**

Man bringt 3,40 g (12,67 mmol) des Olefins in 75 ml Dimethylsulfoxid in Lösung, versetzt anschließend mit 5 ml Wasser und gibt unter kräftigem Rühren 2,80 g N-Bromsuccinimid (15,75 mmol) in einer Portion zu. Zur Aufarbeitung nach 4,5 Stunden Reaktion bei Raumtemperatur gießt man die Reaktionslösung auf Wasser, extrahiert mit Essigester (300 ml), wäscht die organische Phase zunächst mit Wasser, dann mit Sole und trocknet über Natriumsulfat. Das Rohprodukt wird an Kieselgel chromatografiert (Toluol/Aceton, 9:1), Aus-beute 3,50 g (75%) als Öl.

**3-Methoxy-8α-estra-1,3,5(10)-trien-16β-ol**

Eine Lösung von 3,50 g (9,60 mmol) 17α-Brom-3-methoxy-8α-estra-1,3,5(10)-trien-16β-ol, 3,50 g (12,03 mmol) Tributylzinnhydrid und 50 mg Azobisisobutyronitril in 30 ml trockenem Tetrahydrofuran erhitzt man unter Rühren in einer Argonatmosphäre 2 Stunden lang am Rückfluß. Zur Aufarbeitung läßt man abkühlen, engt am Rotavapor im Vakuum ein und nimmt den Rückstand in Essigester (300 ml) auf. Nach Waschen der organischen Phase mit wässriger Salzsäure, Wasser und Sole wird über Natriumsulfat getrocknet. Der Rückstand wird an Kieselgel chromatografiert (Dichlormethan/Essigester, 9:1), Ausbeute 2,70 g (98 %).

**8α-Estra-1,3,5(10)-trien-3,16β-diol (1)**

Eine Lösung von 1,10 g (3,80 mmol) des Methylethers in 35 ml Diisobutylalumiumhydrid/Toluol (1,2 molare Lösung) wird unter einer Argonatmosphäre bei Ausschluß von Feuchtigkeit 4 Stunden lang am Rückfluß erhitzt. Anschließend kühlt man das Reaktionsgemisch im Eisbad ab und versetzt unter Rühren vorsichtig mit Essigester/Wasser. Den entstehenden Niederschlag trennt man durch Filtration ab, wäscht gründlich mit Essigester nach und konzentriert die organische Phase im Vakuum. Das Rohprodukt wird aus Aceton/Hexan umkristallisiert, Ausbeute 679 mg (65 %), Schmelzpunkt 181-182 °C, Drehwert $[\alpha]_D$ +13,6° (c 0,52, $CH_3OH$).

**Beispiel 2**

**8α-Estra-1,3,5(10)-trien-3,16α-diol**

**3-Methoxy-8α-estra-1,3,5(10)-trien-16α-ol**

Zu einer Mischung aus 1,50 g (5,24 mmol) 3-Methoxy-8α-estra-1,3,5(10)-trien-16β-ol, 2,06 g (7,85 mmol) Triphenylphosphin und 0,3 ml Ameisensäure in 10 ml Toluol tropft man unter Rühren 1,22 ml (7,85 mmol) Azodicarbonsäurediethylester gelöst in 2 ml Toluol langsam zu. Anschließend läßt man zwei Stunden bei Raumtemperatur reagieren. Zur Aufarbeitung nimmt man in Essigester (300 ml) auf, wäscht die organische Phase mit Wasser/Sole und

trocknet über Natriumsulfat. Das Rohprodukt wird an Kieselgel chromatografiert (Hexan/ Aceton, Gradient bis 4:1). Man erhält 1,40 g 16α-Formiat, das zur Verseifung in 50 ml 3 %iger methanolischer Kalilauge gelöst wird. Nach einer Stunde bei Raumtemperatur versetzt man mit wässriger Salzsäure, nimmt in Essigester (300 ml) auf, wäscht die organische Phase mit Wasser/Sole und trocknet über Natriumsulfat. Das Rohprodukt wird an Kieselgel chromatografiert (Dichlormethan/ Essigester, Gradient bis 7:3), Ausbeute 940 mg (63 %).

**8α-Estra-1,3,5(10)-trien-3,16α-diol (2)**

Eine Lösung von 740 mg (2,58 mmol) Methylether in 25 ml Diisobutylaluminiumhydrid/Toluol (1,2 molare Lösung) wird in einer Argonatmosphäre unter Feuchtigkeitsausschluß 4 Stunden am Rückfluß erhitzt (130 °C Badtemperatur). Anschließend kühlt man das Reaktionsgemisch im Eisbad ab und versetzt vorsichtig mit Essigester/Wasser. Den Niederschlag trennt man durch Filtration ab, wäscht gründlich mit Essigester nach und konzentriert die organische Phase im Vakuum. Das Rohprodukt wird aus Aceton/Hexan umkristallisiert, Ausbeute 323 mg (46 %), Schmelzpunkt 239-240 °C, Drehwert $[\alpha]_D$ +19,8° (c 0,52, $CH_3OH$).

**Beispiel 3**

**<u>7α-Methylestra-1,3,5(10)-trien-3,16β-diol</u>**

**3-Methoxy-7α-methylestra-1,3,5(10)-trien-17-on-17-phenylsulfonylhydrazen**

Aus 4,70 g (15,75 mmol) 3-Methoxy-7α-methylestra-1,3,5(10)-trien-17-on ergaben sich 4,70 g (66 %) des entsprechenden Phenylsulfonylhydrazons, das beim Abkühlen der Reaktionsmischung auskristallisierte, Schmelzpunkt 167-170 °C.

**3-Methoxy-7α-methylestra-1,3,5(10),16-tetraen**

Aus der Olefinierung von 4,40 g (9,72 mmol) Phenylsulfonylhydrazon resultierten 2,35 g (85 %) Olefin, das aus Ethanol in weißen Schuppen nach Chromatografie an Kieselgel (Hexan/Essigester, 9:1) kristallisierte, Schmelzpunkt 114-116 °C.

**17α-Brom-3-methoxy-7α-methylestra-1,3,5(10)-trien-16β-ol**

Die Bromhydrinbildung mit 2,00 g (7,08 mmol) Olefm erbrachte 2,14 g (80 %) Addukt, Schmelzpunkt 145-146 °C (Ether/Pentan), unter Zersetzung.

**3-Methoxy-7α-methylestra-1,3,5(10)-trien-16β-ol**

Aus 1,94 g (5,12 mmol) Bromid wurden durch reduktive Dehalogenierung 1,40 g (91 %) Produkt, amorph, gewonnen.

**7α-Methylestra-1,3,5(10)-trien-3,16β-diol (3)**

Die Spaltung von 1,40 g (4,66 mmol) Methylether lieferte 1,25 g (92 %) des Diols, dessen Schmelzpunkt bei 209-210 °C (Aceton/Hexan) lag, $[\alpha]_D$ +73,8° (c 0,50, $CH_3OH$).

**Beispiel 4**

**<u>7α-Methylestra-1,3,5(10)-trien-3,16α-diol (4)</u>**

Aus 0,74 g (2,58 mmol) 3,16β-Diol konnten durch Epimerisierung/Verseifung an C(16) 0,434 g (59 %) des 16α-Derivates erhalten werden, Schmelzpunkt 217-219 °C (Aceton/Hexan), $[\alpha]_D$ +84,4° (c 0,52, $CH_3OH$).

**Beispiel 5**

**1-Methoxyestra-1,3,5(10)-trien-3,16β-diol**

**1,3-Dimethoxyestra-1,3,5(10)-trien-17-on-17-phenylsulfonylhydrazon**

3,14 g (10 mmol) 1,3-Dimethoxyestra-1,3,5(10)-trien-17-on ergaben nach der allgemeinen Vorschrift zur Hydrazonbildung 4,0 g (85 %) des 17-Benzolsulfonsäurehydrazons, das aus der ethanolischen Reaktionslösung auskristallisierte, Schmelzpunkt 200-202 °C.

**1,3-Dimethoxyestra-1,3,5(10),16-tetaen**

Die Olefinierung von 4,0 g (8,54 mmol) Hydrazon resultierte in 1,96 g (76 %) Tetraen, das nach Chromatografie aus Ethanol umkristallisiert wurde, Schmelzpunkt 109-111 °C.

**1,3-Dimethoxyestra-1,3,5(10)-trien-16β-ol**

Aus 1,50 g (5,03 mmol) des Olefins wurden durch Bromhydrin-Bildung und Dehalogenierung 0,872 g (55 %) des 16β-Alkohols gewonnen.

**1,3-Dimethoxyestra-1,3,5(10)-trien-16α-ol**

Die Inversion von 0,50 g (1,58 mmol) 16β-Alkohol lieferte 0,46 g (92 %) des 16α-Epimeren.

**1-Methoxyestra-1,3,5(10)-trien-3,16β-diol (5)**

0,25 g (0,79 mmol) 1,3-Dimethoxy-Derivat wurden zu 0,18 g (75 %) Methoxydiol monodemethyliert. Schmelzpunkt nach Verreiben in Toluol 90-93 °C.

**Beispiel 6**

**1-Methoxyestra-1,3,5(10)-trien-3,16α-diol (6)**

Die Demethylierung von 0,35 g (1,11 mmol) Dimethoxy-Derivat in der 16α-Reihe erbrachte 0,218 g (65 %) Monomethylether, Schmelzpunkt 240-242 °C (Aceton/Chloroform).

**Beispiel 7**

**3,11β,16β-Trihydroxyestra-1,3,5(10)-trien-11-nitratester**

**3,16β-Diacetyloxyestra-1,3,5(10)-trien**

Man legt 8,00 g (29,4 mmol) Estra-1,3,5(10)-trien-3,16β-diol bei Raumtemperatur in 50 ml Pyridin vor, versetzt unter Rühren mit 10 ml Essigsäureanhydrid und läßt anschließend über Nacht reagieren. Zur Aufarbeitung trägt man das Reaktionsgemisch in Eiswasser (3 l) ein, wobei das Reaktionsprodukt als Niederschlag ausfällt. Dieser wird auf einer Fritte gesammelt, gründlich mit dest. Wasser gewaschen, getrocknet und schließlich in Dichlor-methan (500 ml) aufgenommen. Die organische Phase wird mit verdünnter Bicarbonat-Lösung und Wasser gewaschen und über Natriumsulfat getrocknet. Der organische Rück-stand wird aus Aceton/Hexan umkristallisiert, Ausbeute 8,40 g (80 %).

**3,16β-Diacetyloxy-9,11β-dihydroxyestra-1,3,5(10)-trien-11-nitratester**

Eine Suspension von 7,85 g (22,0 mmol) 3,16β-Diacetyloxyestra-1,3,5(10)-trien in 200 ml wässriger Essigsäure (90 %ig) wird unter Rühren innerhalb von zehn Minuten portionsweise mit 60,31 g (110 mmol) Cerammonnitrat versetzt. Das steroidale Edukt geht im Verlauf der Reaktion in Lösung. Nach fünf Stunden gießt man die Reaktionslösung auf Eiswasser (6 1) und saugt den gelbrot-gefärbten Niederschlag ab, der anschließend an der Luft getrocknet wird. Das Rohprodukt wird dann in Essigester (600 ml) aufgenommen, die organische Phase mit Wasser/Sole gewaschen und über Natriumsulfat getrocknet. Das rotbraun-gefärbte Rohprodukt wird an Kieselgel chromatografiert (Dichlormethan/Essigester, 9:1), Ausbeute 5,10 g (53 %), Schmelzpunkt 173-175 °C (Aceton/Hexan).

**3,16β-Diacetyloxyestra-1,3,5(10)-trien-11β-ol-11-nitratester**

Zu einer auf -15 °C gekühlten Lösung von 2,42 g (5,58 mmol) 3,16β-Diacetyloxyestra-1,3,5(10)-trien-9,11β-diol-11-nitratester und 2,90 ml (18,31 mmol) Triethylsilan in 60 ml trockenem Dichlormethan tropft man unter Rühren 5,0 ml Bortrifluorid-Etherat. Man läßt zunächst eine Stunde bei -15 °C, dann noch eine weitere Stunde bei 0 °C reagieren bevor man die Reaktionsmischung in bicarbonathaltiges Eiswasser einrührt. Das Produktgemisch wird mit Dichlormethan extrahiert, die organische Phase mit Wasser gewaschen und über Natriumsulfat getrocknet. Das Rohprodukt wird an Kieselgel chromatografiert (Hexan/-Essigester, Gradient bis 7:3). Ausbeute 1,58 g (68 %), Schmelzpunkt 188-190 °C (Aceton/Hexan).

**3,11β,16β-Trihydroxyestra-1,3,5(10)-trien-11-nitratester (7)**

1,31 g (3,14 mmol) des oben beschriebenen Diacetates werden in 60 ml Dichlormethan aufgenommen, mit 20 ml methanolischer Kalilange (3 %ig) versetzt und in einer Schutzgasatmosphäre (Argon) vier Stunden gerührt. Zur Aufarbeitung versetzt man mit 500 µl Essigsäure, verdünnt mit Dichlormethan, wäscht die organische Phase mit Wasser und trocknet über Natriumsulfat. Das Rohprodukt wird aus Dichlormethan umkristallisiert, Ausbeute 874 mg (83 %), Schmelzpunkt 170-171 °C, unter Zersetzung- $[\alpha]_D$ +68,9° (c 0,52, $CH_3OH$).

**Beispiel 8**

**3,11β,16α-Trihydroxyestra-1,3,5(10)-trien-11-nitratester (8)**

Zu einer Lösung von 820 mg (2,46 mmol) 3,16β-Diol in 25 ml trockenem Tetrahydrofuran gibt man 2,26 g (8,62 mmol) Triphenylphosphin und 325 µl Ameisensäure. Anschließend tropft man in diese Lösung unter Rühren langsam 1,34 ml (8,62 mmol) Azodicarbonsäurediethylester bei Raumtemperatur zu. Nach vollendeter Zugabe rührt man noch 30 Minuten bei Raumtemperatur bevor man die Reaktionslösung auf Wasser gießt und mit Essigester extrahiert. Die organische Phase wird mit Wasser/Sole gewaschen und über Natriumsulfat getrocknet. Das Rohprodukt wird in 20 ml Dichlormethan aufgenommen, mit 10 ml methanolischer Kalilauge (3 %ig) versetzt und unter Ausschluß von Luftsauerstoff 2,5 Stunden bei Raumtemperatur gerührt. Zur Aufarbeitung säuert man mit verdünnter Salzsäure an, extrahiert mit Dichlormethan (200 ml), wäscht mit Wasser und trocknet über Natriumsulfat. Das Rohprodukt wird an Kieselgel chromatografiert (Dichlormethan/Aceton, Gradient bis 4:1), Ausbeute 704 mg (85 %) als Schaum. $[\alpha]_D$ +71,4° (c 0,50, $CH_3OH$).

**Beispiel 9**

**18a-Homoestra-1,3,5(10)-trien-3,16α-diol**

**3-Methoxy-18a-homoestra-1,3,5(10)-trien-3,16α-diol**

7,41 g 3-Methoxy-18a-homoestra-1,3,5(10),16-tetraen werden unter Schutzgas in 50 ml wasserfreiem Tetrahydrofuran gelöst und mit 6,4 g 9-Borabicyclo[3.3.1]nonan versetzt. Es wird bei Raumtemperatur bis zur vollständigen Umsetzung gerührt. Anschließend versetzt man mit 75 ml Wasser. Nach beendeter Gasentwicklung werden 45 ml 3 M Natriumhydroxidlösung zugesetzt. In das Reaktionsgemisch werden dann unter Kühlung 45 ml Wasserstoffperoxidlösung (30%) langsam eingetropft. Man rührt 1 h bei Raumtemperatur und extrahiert mit Ethylacetat. Die Chromatographie des Rohproduktes an Kieselgel (Eluent: Cyclohexan/Ethylacetat 3+1) ergibt 6,03 g 18a-Homoestra-1,3,5(10)-trien-3,16α-diol. Nach Umkristallisation aus Methanol erhält man farblose Kristalle; Fp. 109 ... 111 °C; $[\alpha]_D$ =+71 ° (Chloroform, c = 1,02).

**18a-Homoestra-1,3,5(10)-trien-3,16α-diol (9)**

2 g 3-Methoxy-18a-homoestra-1,3,5(10)-trien-16α-ol werden unter Schutzgas in 40 ml Toluol suspendiert. Zu dieser Suspension tropft man 26 ml einer Lösung von Diisobutylaluminiumhydrid (30 Vol.-%) in Toluol und erhitzt bis zur vollständigen Umsetzung am Rückfluß (ca. 10 h). Zum abgekühlten Ansatz werden 10,6 ml Ethanol und unter Kühlung vorsichtig 32 ml halbkonzentrierte Salzsäure gegeben. Nach Extraktion mit Ethylacetat erhält man 1,85 g rohes 18a-Homoestra-1,3,5(10)-trien-3,16α-diol. Die Umkristallisation aus Ethylacetat liefert 1,34 g farblose Kristalle; Fp. 194 ... 198 °C; $[\alpha]_D$ = +69 ° (Dioxan, c = 0,99).

**Beipiel 10**

**18a-Homoestra-1,3,5(10)-trien-3,16β-diol (10)**

0,5 g 18a-Homoestra-1,3,5(10)-trien-3,16α-diol werden in 25 ml Toluol unter Zusatz von 3,66 g Triphenylphosphin und 3,42 g 4-Nitrobenzoesäure gelöst. Dazu werden langsam 6,4 ml Diethylazodicarboxylatlösung (40 % in Toluol) getropft. Nach 48-stündiger Reaktion bei Raumtemperatur verdünnt man mit Ethylacetat und wäscht die organische Phase mit Natriumhydrogencarbonatlösung, Wasser und Natriumchloridlösung. Es wird über Magnesiumsulfat getrocknet und eingeengt.
Das erhaltene Produkt wird in 30 ml Methanol gelöst und mit 4,82 g Kaliumcarbonat versetzt. Es wird bei Raumtemperatur bis zur vollständigen Verseifung gerührt. Zur Aufarbeitung wird die Hauptmenge des Methanols abdestilliert und der Rückstand in Ethylacetat aufgenommen. Man wäscht mit Natriumchloridlösung und trocknet über Magnesiumsulfat. Nach dem Einengen werden 0,45 g rohes 18a-Homoestra-1,3,5(10)-trien-3,16β-diol erhalten. Die Umkristallisation aus Ethylacetat ergibt 0,26 g farblose Kristalle; Fp. 210 ... 213 °C; $[\alpha]_D$ = +67 ° (Dioxan, c = 1,01).

**Beispiel 11**

**18a-Homoestra-1,3,5(10),9(1 1)-tetraen-3,16α-diol**

**18a-Homoestra-1,3,5(10)-trien-3,16α-diyldiacetat**

1 g 18a-Homoestra-1,3,5(10)-trien-3,16α-diol werden mit 4 ml Pyridin und 4 ml Acetanhydrid sowie 10 mg 4-Dimethylaminopyridin versetzt und über Nacht stehen gelassen. Zur Aufarbeitung wird mit Eis versetzt und mit Ethylacetat extrahiert. Die vereinigten Extrakte werden mit Kupfersulfatlösung (10 %) und gesättigter Natriumchloridlösung gewaschen sowie über Magnesiumsulfat getrocknet. Es werden 1,32 g 18a-Homoestra-1,3,5(10)-trien-3,16α-diyldiacetat in Form eines farblosen Schaumes isoliert; $[\alpha]_D$ = +50 ° (Chloroform, c= 1,08).

**18a-Homoestra-1,3,5(10),9(11)-tetraen-3,16α-diyldiacetat**

Zu einer Mischung bestehend aus 12 ml Methylenchlorid, 18 ml Wasser, 15 ml Aceton, 5,4 g Natriunhydrogencarbonat sowie 12 mg Tetra-n-butylammoniumhydrogensulfat werden 1,32 g 18a-Homoestra-1,3,5(10)-trien-3,16a-diyldiacetat gegeben. Nach Temperierung auf 10°C wurden 11,1 g Kaliummonopersulfat (Caroat®) sukzessive zugegeben. Das Reaktionsgemisch wurde 4,5 h bei 10 °C grührt. Danach wurde zur Abtrennung der Salze über eine Fritte filtriert und die organische Phase des Filtrates abgetrennt. Die wäßrige Phase wird mit Methylenchlorid nachextrahiert und die vereinigten Extrakte werden über Magnesiumsulfat getrocknet. Nach Verdampfen des Lösungsmittels werden 1,73 g rohes 9α-Hydroxy-18a-homoestra-1,3,5(10)-trien-3,16α-diyldiacetat erhalten. Dieses wird in 12 ml Methylenchlorid gelöst. Die Lösung wird auf -10 °C temperiert und mit 0,16 ml Schwefelsäure (70 %) vesetzt. Nach beendeter Umsetzung wird mit gesättigter Natriumhydrogencarbonat versetzt und die organische Phase abgetrennt. Es werden nach Trocknung und Verdampfen des Lösungsmittels 1,33 g eines braunen Öles erhalten. Die Reinigung an Kieselgel (Eluent: Cyclohexan/Ethylacetat 3+1) ergibt 0,63 g 18a-Homoestra-1,3,5(10),9(11)-tetraen-3,16α-diyldiacetat als farblosen Schaum; $[\alpha]_D$ = +123° (Chloroform; c = 1,02).

**18a-Homoestra-1,3,5(10),9(11)-tetraen-3,16α-diol (11)**

0,63 g 18a-Homoestra-1,3,5(10),9(11)-tetraen-3,16α-diyldiacetat werden in 50 ml Methanol gelöst und mit 4,72g Kaliumcarbonat versetzt. Es wird bei Raumtemperatur bis zur vollständigen Verseifung gerührt. Zur Aufarbeitung wird die Hauptmenge des Methanols abdestilliert und der Rückstand in Ethylacetat aufgenommen. Man wäscht mit Natriumchloridlösung und trocknet über Magnesiumsulfat. Nach dem Einengen werden 0,49 g 18a-Homoestra-1,3,5(10),9(11)-tetraen- 3, 16α-diol als gelbliches Kristallisat erhalten; Fp. 196 ... 202 °C; $[\alpha]_D$ = +163° (Dioxan; c = 1)

**Beispiel 12**

**Estra-1,3,5(10),9(11)-tetraen-3,16α-diol**

**Estra-1,3,5(10),9(11)-tetraen-3,16α-diyldiacetat**

Analog zu Beispiel 11 werden ausgehend von 19,9 g (55,82 mmol) Estra-1,3,5(10)-trien-3,16α-diyldiacetat 12,69 g (35,8 mmol; 64% d. Th.) Estra-1,3,5(10),9(11)-tetraen-3,16α-diyldiacetat gewonnen.

**Estra-1,3,5(10),9(11)-tetraen-3,16α-diol (12)**

Analog zu Beispiel 11 werden 12,5 g (35,26 mmol) Estra-1,3,5(10),9(11)-tetraen-3,16α-diyldiacetat verseift. Es werden 9,53 g (35,26 mmol; 99 % d. Th.) Estra-1,3,5(10),9(11)-tetraen-3,16α-diol als nahezu farbloses Kristallisat erhalten. Umkristallisation aus Ethylacetat liefert farblose Kristalle; Fp. 237 .. 244 °C; $[\alpha]_D$ = +163 ° (Dioxan; c = 1,12)

**Beispiel 13**

<u>**13α-Estra-1,3,5(10)-trien-3,16α-diol**</u>

**3-Methoxy-17-tosylhydrazono-13α-estra-1,3,5(10)-trien**

2,5 g (8,79 mmol) 3-Methoxy-13α-estra-1,3,5(10)-trien-17-on und 1,96 g (10,55 mmol) Tosylhydrazid werden in 15 ml eines Gemisches aus Ethanol und Eisessig (4+1, v/v) 6 h am Rückfluß erhitzt Die abgekühlte Reaktionslösung wird mit gesättigter Natriumhydrogencarbonatlösung versetzt und mit Ethylacetat extrahiert. Die organische Phase wird mit gesättigter Natriumhydrogencarbonatlösung und mit gesättigter Natriumchloridlösung gewaschen sowie über Magnesiumsulfat getrocknet. Das erhaltene dunkelbraune Öl wird an Kieselgel choramtographiert (Eluent: Cyclohexan/Ethylacetat 4 + 1). Man erhält 2,49 g eines farblosen, amorphen Festoffes; $[\alpha]_D$= -58 ° (Dioxan, c = 0,99).

**3-Methoxy-13α-estra-1,3,5(10),16-tetraen**

2,43 g (5,37 mmol) 3-Methoxy-17-tosylhydrazono-13α-estra-1,3,5(10)-trien werden im 20 ml wasserfreiem Methyl-tert.-butylether suspendiert. Zu dieser Suspension werden langsam 1,61 ml einer 10 M n-Butyllithiumlösung in Hexan getropft. Es wird 1 h bei RT gerührt. Unter Kühlung werden 50 ml gesättigte Ammoniumchloridlösung zugetropft. Nach Abtrennung der organischen Phase extrahiert man mit Ethylacetat. Die vereinigten organischen Phasen werden mit Wasser und gesättigter Natriumchloridlösung gewaschen und getrocknet ($MgSO_4$). Das Rohprodukt (2,1 g braunes Öl) wird an Kieselgel chromatographiert wobei 0,81 g 3-Methoxy-13α-estra-1,3,5(10),16-tetraen als farbloses Öl erhalten werden; $[\alpha]_D$= -6 ° (Chloroform; c = 0,94).

**3-Metboxy-13α-estra-1,3,5(10)-trien-16α-ol**

0,81 g (3 mmol) 3-Methoxy-13α-estra-1,3,5(10),16-tetraen werden unter Schutzgas in 10 ml wasserfreiem Tetrahydrofuran gelöst und mit 0,73 g 9-Borabicyclo[3.3.1]nonan versetzt. Es wird bei Raumtemperatur bis zur vollständigen Umsetzung gerührt. Anschließend versetzt man mit 15 ml Wasser. Nach beendeter Gasentwicklung werden 7,8 ml 3 M Natriumhydroxidlösung zugesetzt. In das Reaktionsgemisch werden dann unter Kühlung 7,8 ml Wasserstoffperoxidlösung (30%) langsam eingetropft. Man rührt 1 h bei Raumtemperatur und extrahiert mit Ethylacetat. Die Chromatographie des Rohproduktes an Kieselgel (Eluent: Cyclohexan/Ethylacetat 4+1) ergibt 604 mg 3-Methoxy-13α-estra-1,3,5(10)-trien-16α-ol in Form eines farblosen Öles.

**13α-Estra-1,3,5(10)-trien-3,16α-diol (13)**

0,55 g 3-Methoxy-13α-estra-1,3,5(10)-trien-16α-ol werden unter Schutzgas in 10 ml Toluol heiß gelöst. Zu dieser Lösung tropft man eine Mischung aus 2,3 ml Diisobutylaluminiumhydrid und 5,4 ml Toluol und erhitzt bis zur vollständigen Umsetzung am Rückfluß (ca. 4 h). Zum abgekühlten Ansatz werden 2,1 ml Ethanol und unter Kühlung vorsichtig 6 ml halbkonzentrierte Salzsäure gegeben. Nach Extraktion mit Ethylacetat wäscht man die organische Phase neutral und trocknet über Magnesiumsulfat. Es werden 362 mg 13α-Estra-1,3,5(10)-trien-3,16α-diol erhalten. Die Umkristallisation aus Mehanol liefert farblose Kristalle; Fp. 224 ... 231 °C; $[\alpha]_D$ =+61 ° (Pyridin, c =1,13).

**Beispiel 14**

<u>**9β-Estra-1,3,5(10)-trien-3,16α-diol**</u>

**3-Methoxy-17-tosylhydrazono-9β-estra-1,3,5(10)-trien**

Analog zu Beispiel 13 dargestellt. Farbloser Schaum.

**3-Methoxy-9β-estra-1,3,5(10),16-tetraen**

Analog zu Beispiel 13 dargestellt. Farbloses Öl.

**3-Methoxy-9β-estra-1,3,5(10)-trien-16α-ol**

Analog zu Beispiel 13 dargestellt. Farbloses Öl.

**9β-Estra-1,3,5(10)-trien-3,16α-diol (14)**

Analog zu Beispiel 13 dargestellt. Farblose Kristalle; Fp. 140 ... 145 °C; $[\alpha]_D$ = -91 ° (Dioxan; c = 0,98).

**Beispiel 15**

**3,16α-Bis(benzyloxy)-18a-homoestra-1,3,5(10)-trien-11-on**

**3,16α-Bis(benzyloxy)-18a-homoestra-1,3,5(10),9(11)-tetraen**

4 g Natriumhydrid (80% in Paraffinöl, 25,52 mmol) werden unter Schutzgas zu 32 ml wasserfreiem N,N-Dimethylformamid gegeben. In dieses Gemisch wird eine Lösung von 7,67 g (26,97 mmol) 18a-Homoestra-1,3,5(10)-trien-3,16α-diol in 40 ml Tetrahydrofuran eingetropft. Nach abgeschlossener Gasentwicklung fügt man 13,84 g (80,91 mmol) Benzylbromid zu. Nach beendeter Umsetzung wird die Reaktionslösung langsam in Wasser (ca. 11) getropft. Nach Extraktion mit Ethylacetat werden 14 g braunes Öl erhalten. Chromatographie an Kieselgel ergibt 10,2 g (21,9 mmol; 81,3 % d.Th.) 3,16α-Bis(benzyloxy)-18a-homoestra-1,3,5(10),9(11)-tetraen als farbloses Öl; $[\alpha]_D$ = +110° (Chloroform, c = 1,01).

**3,16α-Bis(benzyloxy)-18a-homoestra-1,3,5(10)-trien-11α-ol**

10 g (21,5 mmol) 3,16α-Bis(benzyloxy)-18a-homoestra-1,3,5(10),9(11)-tetraen werden unter Schutzgas in 60 ml wasserfreiem Tetrahydrofuran gelöst und 12,9 g (107,61 mmol) Catecholboran sowie 0,99 g (43,1 mmol) Lithiumborhydrid zugegeben. Nach beendeter Umsetzung wird mit 100 ml Wasser vorsichtig hydrolysiert. Es werden dann 95 ml 3N Natronlauge zugesetzt und unter Kühlung 95 ml Wasserstoffperoxid (30%) eingetropft. Es wird 1 h bei Raumtemperatur gerührt und nachfolgend mit Ethylacetat extrahiert. Die Chromatographie des Rohproduktes an Kieselgel (Eluent: Cyclohexan/Ethylacetat 4+1) ergibt 8,73 g (18,08 mmol; 84% d.Th.) 3,16α-Bis(benzyloxy)-18a-homoestra-1,3,5(10)-trien-11α-ol als farblosen Schaum; $[\alpha]_D$ = -48 °(Chloroform; c = 0,96).

**3,16α-Bis(benzyloxy)-18a-homoestra-1,3,5(10)-trien-11-on (15)**

0,89 g (1,84 mmol) 3,16α-Bis(benzyloxy)-18a-homoestra-1,3,5(10)-trien-11α-ol werden in 20 ml Methylenchlorid gelöst und mit 0,98 g (4,6 mmol) Pyridiumchlorochromat versetzt. Es wird 4 h gerührt. Die Reaktionsmischung wird daraufhin mit Tetrachlormethan verdünnt und über Kieselgel filtriert. Das Filtrat wird eingeengt und an Kieselgel chromatographiert (Eluent: Cyclohexan/Ethylacetat 4+1). Es werden 0,63 g (1,31 mmol; 71% d. Th.) 3,16α-Bis(benzyloxy)-18a-homoestra-1,3,5(10)-trien-11-on als farbloser Feststoff erhalten; $[\alpha]_D$ = +177 ° (Chloroform; c = 0,96); IR (ν C=O) = 1705 $cm^{-1}$.

**Beispiel 16**

**3,16α-Bis(benzyloxy)estra-1,3,5(10)-trien-11-on**

**3,16α-Bis(benzyloxy)estra-1,3,5(10),9(1 1)-tetraen**

Analog zu Beispiel 15 werden aus 8,7 g (32,17 mmol) Estra-1,3,5(10),9(11)-tetraen-3,16a-diol 12,83 g (28,47 mmol; 88% d. Th.) 3,16α-Bis(benzyloxy)estra-1,3,5(10),9(11)-tetraen als farbloses Öl gewonnen; $[\alpha]_D$ = +96° (Chloroform, c = 1).

**3,16α-Bis(benzyloxy)estra-1,3,5(10)-trien-11α-ol**

Analog zu Beispiel 15 werden aus 12,74 g (28,27 mmol) 3,16α-Bis(benzyloxy)estra-1,3,5(10),9(1 1)-tetraen

9,43 g (20,2 mmol; 71% d. Th.) 3,16α-Bis(benzyloxy)estra-1,3,5(10)-trien-1α-ol als farbloser Feststoff gewonnen; $[\alpha]_D$ = - 44 ° (Chloroform; c = 1,02).

**3,16α-Bis(benzyloxy)estra-1,3,5(l0)-trien-11-on** (16)

Analog zu Beispiel 15 werden aus 3 g (6,4 mmol) 3,16α-Bis(benzyloxy)estra-1,3,5(10)-trien-11α-ol 1,91 g (4,09 mmol; 64% d. Th.) 3,16α-Bis(benzyloxy)estra-1,3,5(10)-trien-11-on gewonnen; $[\alpha]_D$ =+197 ° (Chloroform; c = 0,96); IR (ν C=0) = 1709 $cm^{-1}$

**Beispiel 17**

**<u>9α-Methyl-18a-homoestra-1,3,5(10)-trien-3,16α-diol</u>**

**3,16α-Bis(benzyloxy)-9α-methyl-18a-homoestra-1,3,5(10)-trien-11-on**

Unter Schutzgas werden 0,8 g (7,13 mmol) Kalium-tert.-butylat in 6,26 ml tert.-Butanol gelöst; 0,8 g (1,66 mmol) 3,16α-Bis(benzyloxy) -18a-homoestra-1,3,5(10)-trien-11-on werden in 15,57 ml Methyliodid gelöst und in die erste Lösung getropft. Nach 15 min werden 50 ml gesättigte Natriumchloridlösung zugesetzt. Die Extraktion mit Ethylacetat liefert einen gelben Schaum, welcher an Kieselgel (Eluent: Cyclohexan/Ethylacetat 7+1) chromatographiert wird. Es werden 0,6 g (1,21 mmol; 73% d. Th.) 3,16α-Bis(benzyloxy)-9α-methyl-18a-homoestra-1,3,5(10)-trien-11-on als farbloser Schaum erhalten; $[\alpha]_D$ = +216° (Chloroform, c = 1,03); IR (ν C=O) = 1705 $cm^{-1}$.

**3,16α-Bis(benzyloxy)-9α-methyl-18a-homoestra-1,3,5(10)-trien**

0,76 g (1,54 mmol) 3,16α-Bis(benzyloxy)-9α-methyl-18a-homoestra-1,3,5(10)-trien-11-on werden mit 5 ml Triethylenglycol in einem Kolben vorgelegt. Zu dieser Mischung gibt man eine Lösung von 2,16 g (38,15 mmol) Kaliumhydroxid in 15 ml Triethylenglycol sowie 1,54 g (30,8 mmol) Hydrazinhydrat. Dieses Gemisch wird 4 h auf 210 °C erhitzt. Anschließend versetzt man mit gesättigter Natriumchloridlösung und extrahiert mit Ethylacetat. Die organischen Phasen werden mit verdünnter Salzsäure, Wasser und Natriumchloridlösung gewaschen. Das Rohprodukt wird an Kieselgel chromatographiert (Eluent: Cyclohexan/Ethylacetat 14+1). Dabei fallen 0,66 g (1,37 mmol; 89% d. Th.) 3,16α-Bis(benzyloxy)-9α-methyl-18a-homoestra-1,3,5(10)-trien als farbloses Öl an; $[\alpha]_D$ =+47° (Chloroform, c = 0,93).

**9α-Methyl-18a-homoestra-1,3,5(10)-trien-3,16α-diol (17)**

0,65 g (1,37 mmol) 3,16α-Bis(benzyloxy)-9α-methyl-18a-homoestra-13,5(10)-trien werden in 20 ml Tetrahydrofuran gelöst, mit 0,65 g Palladium-Kohle (10% Pd) vesetzt und hydriert. Der Katalysator wird abfiltriert und das Filtrat eingeengt, wobei 0,4 g (1,33 mmol; 97% d.Th.) 9α-Methyl-18a-homoestra-1,3,5(10)-trien-3,16α-diol zurückbleiben. Umkristallisation aus Methanol liefert farblose Kristalle; Fp. 210 ... 215 °C; Fp. 210 ... 215 °C; $[\alpha]_D$ = +72 ° (Dioxan; c = 0,99).

**Beispiel 18**

**<u>9α-Methylestra-1,3,5(10)-trien-3,16α-diol</u>**

**3,16α-Bis(benzyloxy)-9α-methylestra-1,3,5(10)-trien-11-on**

Analog zu Beispiel 17 werden aus 0,497 g (1,06 mmol) 3,16α-Bis(benzyloxy)estra-1,3,5(10)-trien-11-on 0,379 g (0,78 mmol; 73% d.Th.) 3,16α-Bis(benzyloxy)-9α-methylestra-1,3,5(10)-trien-11-on als farbloser Schaum gewormen; $[\alpha]_D$ = +231° (Chloroform, c = 1.03); IR (ν C=O) = 1709 $cm^{-1}$.

**3,16α-Bis(benzyloxy)-9α-methylestra-1,3,5(10)-trien**

Analog zu Beispiel 17 werden aus 0,715 g (1,48 mmol) 3,16α-Bis(benzyloxy)-9α-methylestra-1,3,5(10)-trien-11-on 0,458 g (0,98 mmol; 66% d. Th.) 3,16α-Bis(benzyloxy)-9α-methylestra-1,3,5(10)-trien als farbloses Öl gewonnen; $[\alpha]_D$ = +61 ° (Chloroform, c = 1,16).

**9α-Methylestra-1,3,5(10)-trien-3,16α-diol (18)**

Analog zu Beispiel 17 werden aus 0,476 g (1,01 mmol) 3,16α-Bis(benzyloxy)-9α-methylestra-1,3,5(10)-trien 0,292 g (1 mmol; 99% d.Th.) 9α-Methylestra-1,3,5(10)-trien-3,16α-diol gewonnen. Die Umkristallisation liefert farblose Kristalle; Fp. 182 ... 186 °C; $[\alpha]_D$ = +77 ° (Dioxan; c = 0,99).

**Beispiel 19**

**11β-Methyl-18a-homoestra-1,3,5(10)-trien-3,16α-diol**

**3,16α-Bis(benzyloxy)-11α-methyl-18a-homoestra-1,3,5(10)-trien-11β-ol**

0,6 g (1,25 mmol) 3,16α-Bis(benzyloxy)-18a-homoestra-1,3,5(10)-trien-11-on werden unter Schutzgas in 20 ml wasserfreiem Tetrahydrofuran gelöst. Diese Lösung wird auf -15°C gekühlt und mit 4,17 ml 3M Methylmagnesiumbromidlösung versetzt. Nach vollständiger Umsetzung wird gesättigte Ammoniumchloridlösung zugegeben und mit Ethylacetat extrahiert. Das Rohprodukt wird an Kieselgel (Eluent: Cyclohexan/Ethylacetat 6+1) chromatographiert, wobei 0,59 g (1,18 mmol; 95% d.Th.) 3,16α-Bis(benzyloxy)-11α-methyl-18a-homoestra-1,3,5(10)-trien-11β-ol als farbloses Öl anfallen; $[\alpha]_D$ = 1° (Chloroform, c = 0,99).

**3,16α-Bis(benzyloxy)-11β-methyl-18a-homoestra-1,3,5(10)-trien**

0,5 g(1 mmol) 3,16α-Bis(benzyloxy)-11α-methyl-18a-homoestra-1,3,5(10)-trien-11β-ol werden unter Schutzgas in Methylenchlorid gelöst und mit 1,75 g (2,4 ml; 15,3 mmol) Triethylsilan versetzt. Es wird auf -10°C gekühlt und mit 5,69 g (5 ml, 40 mmol) Bortrifluoridethyletherat versetzt. Nach beendeter Umsetzung wird mit gesättigter Natriumhydrogencarbonatlösung versetzt und extrahiert. Das Rohprodukt wird an Kieselgel (Eluent: Cyclohexan/Ethylacetat 9+1) gereinigt. Es werden 0,327 g (0,68 mmol; 68% d. Th.) 3,16α-Bis(benzyloxy)-11β-methyl-18a-homoestra-1,3,5 (10)-trien als farbloses Öl isoliert; $[\alpha]_D$ = +119° (Chloroform, c = 0,99).

**11β-Methyl-18a-homoestra-1,3,5(10)-trien-3,16α-diol (19)**

0,45 g (0,93 mmol) 3,16α-Bis(benzyloxy)-11β-methyl-18a-homoestra-1,3,5(10)-trien werden in 20 ml Tetrahydrofuran gelöst, mit 0,45 g Palladium-Kohle (10% Pd) vesetzt und hydriert. Der Katalysator wird abfiltriert und das Filtrat eingeengt, wobei 0,264 g (0,87 mmol; 94% d.Th.) 11β-Methyl-18a-homoestra-1,3,5(10)-trien-3,16α-diol zurückbleiben. Umkristallisation aus Ethylacetat liefert farblose Kristalle; Fp. 244 ... 251 °C; $[\alpha]_D$ = +197 ° (Dioxan; c = 1,07).

**Beispiel 20**

**11 β-Methyl-18a-homoestra-1,3,5(10)-trien-3,16β-diol (20)**

0,1 g (0,33 mmol) 11β-Methyl-18a-homoestra-1,3,5(10)-trien-3,16α-diol werden in Toluol unter Zusatz von 0,35 g (1,33 mmol) Triphenylphosphin und 0,22 g (1,33 mmol) 4-Nitrobenzoesäure gelöst. Dazu werden langsam 0,6 ml (1,33 mmol) Diethylazodicarboxylatlösung (40 % in Toluol) getropft. Es wird bis zur vollständigen Umsetzung auf 50 °C erwärmt. Danach verdünnt man mit Ethylacetat und wäscht die organische Phase mit Natriumhydrogencarbonatlösung, Wasser und Natriumchloridlösung. Es wird über Magnesiumsulfat getrocknet und eingeengt.
Das erhaltene Produkt wird in 20 ml Methanol gelöst und mit 0,81 g (5,85 mmol) Kaliumcarbonat versetzt. Es wird bei Raumtemperatur bis zur vollständigen Verseifung gerührt. Zur Aufarbeitung wird die Hauptmenge des Methanols abdestilliert und der Rückstand in Ethylacetat aufgenommen. Man wäscht mit Natriumchloridlösung und trocknet über Magnesiumsulfat. Das Rohprodukt wird an Kieselgel (Eluent: Cyclohexan/Ethylacetat 2+1) chromatographiert, wobei 0,79 g (0,26 mmol; 78% d. Th.) 11β-Methyl-18a-homoestra-1,3,5(10)-trien-3,16β-diol gewonnen werden. Die Umkristallisation aus Ethylacetat ergibt farblose Kristalle; Fp. 175 ... 188 °C; $[\alpha]_D$ = +148 ° (Dioxan; c = 0,95).

**Beispiel 21**

**11 β-Methylestra-1,3,5(10)-trien-3,16α-diol**

**3,16α-Bis(benzyloxy)-11α-methylestra-1,3,5(10)-trien-11β-ol**

Analog zu Beispiel 19 dargestellt. Farbloses Öl; $[\alpha]_D$ = +2° (Chloroform, c = 0,92).

**3,16α-Bis(benzyloxy)-11 β-methylestra-1,3,5(10)-trien**

Analog zu Beispiel 19 dargestellt. Farbloses Öl; $[\alpha]_D$ =+112° (Chloroform, c = 1).

**11β-Methylestra-1,3,5(10)-trien-3,16α-diol (21)**

Analog zu Beispiel 19 dargestellt. Farblose Kristalle aus Methyl-tert.-butylether; Fp. 243 ... 250 °C; $[\alpha]_D$ = +172 ° (Dioxan, c = 0,96).

**Beispiel 22**

**11β-Methylestra-1,3,5(10)-trien-3,16β-diol (22)**

Analog zu Beispiel 20 dargestellt. Farblose Kristalle aus Cyclohexan/Ethylacetat; Fp. 194 ... 199 °C; $[\alpha]_D$ = +177 ° (Dioxan, c = 0,97).

**Beispiel 23**

**11 β-Ethyl-18a-homoestra-1,3,5(10)trien-3,16α-diol**

**3,16α-Bis(benzyloxy)-11α-ethyl-18a-homoestra-1,3,5(10)-trien-11β-ol**

Analog zu Beispiel 19 dargestellt. Farbloses Öl.

**3,16α-Bis(benzyloxy)-11β-ethyl-18a-homo estra-1,3,5(10)-trien**

Analog zu Beispiel 19 dargestellt. Farbloses Öl.

**11β-Ethyl-18a-homoestra-1,3,5(10)-trien-3,16α-diol (23)**

Analog zu Beispiel 19 dargestellt. Farblose Kristalle 242 ... 255 °C; $[\alpha]_D$ = +140 ° (Pyridin, c = 0,99).

**Beispiel 24**

**11β-Ethyl-18a-homoestra-1,3,5(10)-trien-3,16β-diol (24)**

Analog zu Beispiel 20 dargestellt. Farblose Kristalle 152 ... 156 °C; $[\alpha]_D$ = +148 ° (Dioxan, c = 0,95).

**Beispiel 25**

**11 β-Ethylestra-1,3,5(10)-trien-3,16α-diol**

**3,16α-Bis(benzyloxy)-11α-ethylestra-1,3,5(10)-trien-11β-0l**

Analog zu Beispiel 19 dargestellt. Farbloses Öl; $[\alpha]_D$ = -3.° (Chloroform, c = 1).

**3,16α-Bis(benzyloxy)-11 β-ethylestra-1,3,5(10)-trien**

Analog zu Beispiel 19 dargestellt. Farbloses Öl; $[\alpha]_D$ = +97 °(Chloroform, c =1).

**11 β-Ethylestra-1,3,5,(10)-trien-3,16α-diol (25)**

Analog zu Beispiel 19 dargestellt. Farblose Kristalle aus Ethylacetat; Fp. 245 ... 250 °C; $[\alpha]_D$ = +104 ° (Dioxan, c = 1).

**Beispiel 26**

**11β-Ethylestra-1,3,5(10)-trien-3,16β-diol (26)**

Analog zu Beispiel 20 dargestellt. Amorpher Feststoff.

**Beispiel 27**

**11β-Methoxy-estra-1,3,5(10)-trien-3,16α-diol**

**11 β-Methoxy-17-tosylhydrazono-estra-1,3,5(10)-trien-3-ol**

In einem Gemisch aus 6 ml Ethanol und 4 ml Eisessig werden 1 g (3,3 mmol) 3-Hydroxy-11β-methoxy-estra-1,3,5(10)-trien-17-on und 0,7 g (3,8 mmol) Toluol-4-sulfonsäurehydrazid bis zum Rückfluß erwärmt. Nach einer Reaktionszeit von ca. 5 Stunden bei Siedehitze wird das Reaktionsgemisch abgekühlt und das Produkt durch Eintropfen in ca. 100 ml Wasser isoliert. Durch Kochen des Rohproduktes in n-Hexan wird das enthaltene Wasser azeotrop entfernt. Man erhält 1,14 g Produkt (73 % d.Th.).

**11β-Methoxy-estra-1,3,5(10),16-tetraen-3-ol**

469 mg (1 mmol) 11β-Methoxy-17-tosylhydrazono-estra-1,3,5(10)-trien-3-ol werden in 15 ml Tetrahydrofuran vorgelegt. Unter Inertgas und starkem Rühren versetzt man die Lösung bei Raumtemperatur mit 1 ml (10 mmol) n-Butyllithiumlösung (10 M, n-Hexan). Die Reaktionslösung erwärmt sich bis zum Rückfluß. Nach ca. 10 min Reaktionszeit und Abkühlung erfolgt die Aufarbeitung durch Zutropfen von 20 ml gesättigter Ammoniumchloridlösung und 30 ml Essigsäureethylester. Die organische Phase wird mit Wasser/Kochsalzlösung gewaschen und über Natriumsulfat getrocknet. Das Rohprodukt reinigt man durch Chromatografie an Kieselgel (Cyclohexan/Essigester, 1:1). Ausbeute 170 mg (60 % d.Th.)

**11β-Methoxy-3-triethylsilyloxy-estra-1,3,5(10),16-tetraen**

284 mg (1 mmol) 11β-Methoxy-estra-1,3,5(10),16-tetraen-3-ol werden in 10 ml *tert*-Butylmethylether und 1 ml Pyridin mit 0,6 ml Triethylbromsilan umgesetzt. Nach 1 Stunde gibt man zur Reaktionssuspension 30 ml Wasser. Die organische Phase wird abgetrennt, gewaschen, getrocknet und Vakuum eingeengt. Das so erhaltene ölige Produkt wird sofort in der nächsten Stufe (Hydroborierung) eingesetzt. Ausbeute 380 mg (95 % d.Th.)

**11β-Methoxy-estra-1,3,5 (10)-trien-3,16α-diol (27)**

380 mg (0,95 mmol) 11 β-Methoxy-3-triethylsilyloxy-estra-1,3,5(10),16-tetraen werden in 20 ml Tetrahydrofuran unter Inertgas gelöst. Nach Zugabe von 464 mg (3,8 mmol) 9-Borablcyclo[3.3. 1.]nonan rührt man die Reaktionslösung bei Raum-temperatur bis zur vollständigen Umsetzung. Anschließend werden 5 ml Wasser und nach beendeter Gasentwicklung 2 ml 5 N Natronlauge sowie 2 ml 30 %-ige Wasser-stoffperoxidlösung zugetropft. Man rührt 1 Stunde bei Raumtemperatur und extra-hiert das entstandene Produkt mit Essigsäureethylester. Das erhaltene Rohprodukt wird durch Chromatografie an Kieselgel (n-Hexan/Chloroform/Methanol 45/45/10) aufgereinigt und aus Essigsäureethylester/n-Hexan kristallisiert. Man erhält farblose Kristalle: 230 mg (80% d.Th.).
Schmelzpunkt 212-222°C; $[\alpha]_D^{20}$ = +101° (Dioxan, c = 0,53)

**Beispiel 28**

**11β-Methoxy-estra-1,3,5(10)-trien-3,16β-diol (28)**

229 mg (0,76 mmol) 11β-Methoxy-estra-1,3,5(10)-trien-3,16α-diol werden in 30ml Toluol zusammen mit 1,8 g (6,8 mmol) Triphenylphosphin und 1,14 g (6,8 mmol) 4-Nitrobenzoesäure gelöst. Zu dieser Lösung gibt man tropfenweise 2,7 ml (6,8 mmol) Azodicarbonsäurediethylester (40% in Toluol). Nach 24-stündiger Reaktion bei Raumtemperatur wurde die Reaktionslösung mit Essigsäureethylester versetzt. Die so erhaltene organische Phase extrahiert man mit Natriumhydrogen-carbonatlösung, Wasser und Natriumchloridlösung. Die organische Phase wird eingeengt und anschließend das Produkt in Methanol aufgenommen. Nach Zugabe von 2,5 g Kaliumcarbonat wird die Suspension unter Rückfluß bis zur vollständigen Verseifung gekocht. Zur Aufarbeitung destilliert man das Methanol ab und nimmt das Rohprodukt in Essigsäureethylester auf, wäscht mit Wasser sowie Natriumchlorid-lösung und engt die Lösung ein.

Nach Umkristallisation aus Chloroform/Cyclohexan erhält man fast farblose Kristalle:
195 mg (85% d.Th.) ; Schmelzpunkt 195-200°C; $[\alpha]_D^{20}$ = +86° (Dioxan, c = 1,18)

**Beispiel 29**

**11β-Phenyl-estra-1,3,5(10)-trien-3,16α-diol**

**11β-Phenyl-17-tosylhydrazono-estra-1,3,5(10)-trien-3-ol**

590 mg (1,71 mmol) werden wie in Beispiel 28 beschrieben mit Toluol-4-sulfonsäurehydrazid umgesetzt. Durch Abkühlen der Reaktionslösung fällt in diesem Fall ein Teil des Produktes aus. Das Festprodukt wird abgesaugt, mit Ethanol ge-waschen und getrocknet. Man erhält 450 mg gelbliches Kristallisat (51 % d.Th.). Das in der Mutterlauge noch enthaltene Produkt kann durch Extraktion und entsprechen-der chromatografischer Aufarbeitung isoliert werden (303 mg, 38 % d.Th.).

**11β-Phenyl-estra-1,3,5(10),16-tetraen-3-ol**

515 mg (1 mmol) 11 β-Phenyl-17-tosylhydrazono-estra-1,3,5(10)-trien-3-ol setzt man wie im Beispiel 28 mit 1 ml (10 mmol) n-Butyllithiumlösung um. Man erhält 450 mg Rohprodukt, das in der nächsten Stufe zum Triethylsilylether umgesetzt wird.

**11β-Phenyl-3-triethylsilyl-estra-1,3,5(10),16-tetraen**

Entsprechend dem Beispiel 28 werden 450 mg 11β-Phenyl-estra-1,3,5(10),16-tetraen-3-ol (Rohprodukt) zum Triethylsilylether umgesetzt. Das erhaltene Produkt wird durch Chromatografie an Kieselgel (Cyclohexan/Essigsäureethylester, 6/1) gereinigt und die Lösung im Vakuum eingeengt. Man erhält eine ölige Substanz. Ausbeute 320 mg (72 % d.Th. bezogen auf 515mg 11β-Phenyl-17-tosylhydrazono-estra-1,3,5(10)-trien-3-ol).

**11β-Phenyl-estra-1,3,5(10)-trien-3,16α-diol (29)**

Die Hydroborierung erfolgt entsprechend Beispiel 27. 320 mg (0,72 mmol) 11β-Phenyl-3-triethylsilyl-estra-1,3,5(10),16-tetraen ergaben nach Aufarbeitung und Reinigung durch Chromatografie an Kieselgel (Toluol/Essigsäureethylester, 70/30) und Kristallisation aus Methanol 183 mg (73 % d.Th.).
Schmelzpunkt 254-261 $[\alpha]_D^{20}$ = -103° (Dioxan, c = 0,09)

**Beispiel 30**

**11β-Phenyl-estra-1,3,5(10)-trien-3,16β-diol (30)**

Die Inversion der 16-Hydroxygruppe wurde entsprechend dem Beispiel 28 durchgeführt. 36 mg (0,1 mmol) 11 β-Phenyl-estra-1,3,5(10)-trien-3,16α-diol ergaben nach Chromatografie an Kieselgel (Toluol/Essigsäureethylester, 70/30) und Kristal-lisation aus Toluol 25 mg (69 % d.Th.) farblose Kristalle.
Schmelzpunkt 241-247° $[\alpha]_D^{20}$ = -93° (Dioxan, c = 0,31)

**Beispiel 31**

**16α-Ethinyl-18a-homo-estra-1,3,5(10)-trien-3,16β-diol**

**3-Hydroxy-18a-homoestra-1,3,5(10)-trien-16-on:**

2,4 g 18a-Homoestra-1,3,5(10)-trien-3,16α-diol werden in 60 ml Aceton unter Zusatz von 2,5 ml Methylenchlorid gelöst. Diese Lösung wird auf 10 °C gekühlt und tropfenweise mit 4,2 ml Chromschwefelsäure (8 mol/l $CrO_3$) versetzt. Nach beendeter Umsetzung wird mit Natriumhydrogensulfitlösung versetzt und die Hauptmenge des Acetones abdestilliert. Zum verbleibenden Rückstand werden ca. 100 ml Wasser gegeben. Anschließend wird das ausgefallenen Steroid abgesaugt. Nach Trocknung erhält man 2,02 g 3-Hydroxy-18a-homoestra-1,3,5(10)-trien-16-on in Form farbloser Kristalle; Fp. 264 ... 266 °C; $[\alpha]_D$ = -115 ° (Pyridin, c = 0,743).

**16α-Ethinyl-18a-homo-estra-1,3,5(10)-trien-3,16β-diol (31)**

Bei einer Temperatur von ca. 0°C werden 30ml Tetrahydrofuran mit Ethin gesättigt. Anschließend gibt man zur Lösung unter Kühlen und Rühren 3,4 ml (8,4 mmol) n-Butyllithiumlösung (2,5 M, Toluol). Die Temperatur sollte ca. 0°C betragen. Der so erhaltenen Suspension von Lithiumacetylid setzt man eine Lösung von 141 mg (0,5 mmol) 3-Hydroxy-18a-homo-estra-1,3,5(10)-trien-16-on in 10 ml Tetrahydro-furan zu. Nach 30 Minuten Reaktionszeit bei ca. 0 °C wird die Reaktionslösung mit verdünnter Salzsäure versetzt. Nach Abdestillieren des Tetrahydrofurans nimmt man den organischen Rückstand in Toluol auf, trennt die Phasen, wäscht die organische Phase mit Wasser und isoliert das Rohprodukt durch Einengen der Lösung unter Vakuum. Eine Reinigung des Produktes wird durch Chromatografie an Kieselgel (Toluol/Aceton, 7/1) und Kristallisation aus Toluol erreicht. Man erhält
131 mg (85 % d.Th.) kristalline Substanz.
Schmelpunkt 197-202 °C $[\alpha]_D^{20}$ = +100° (Dioxan, c = 1,06)

**Beispiel 32**

**<u>16β-Ethinyl-18a-homo-estra-1,3,5(10)-trien-3,16α-diol (32)</u>**

Die Herstellung von 16β-Ethinyl-18a-homo-estra-1,3,5(10)-trien-3,16α-diol wird analog dem Beispiel 31 durchgerührt. Die Erhöhung der Reaktiontemperatur auf Raumtemperatur erbrachte einen größeren Anteil an 16β-Ethinylprodukt. Durch Chromatografie an Kieselgel (Cyclohexan/Essigsäureethylester, 3/1) konnte das Produkt isoliert werden.
Ausbeute 20 % d.Th., 213-219 °C, $[\alpha]_D^{20}$ = +48° (Dioxan, c =1,04)

**Beispiel 33**

**<u>11β-Fluor-7α-methylestra-1,3,5(10)-trien-3,16β-diol</u>**

**<u>11β-Fluor-7α-methylestra-1,3,5(10)-trien-3,16α-diol</u>**

**11 β-Fluor-7α-methylestr-4-en-3,17-dion**

Eine Suspension von 15,2 g (79,8 mmol) Kupferiodid in 70 ml trockenem Tetrahydrofuran kühlt man auf 0 °C, versetzt mit 28,7 g (330 mmol) Lithiumbromid und 27,8 ml DMPU, rührt zunächst 30 Minuten bei dieser Temperatur und kühlt anschließend auf-30 °C. Unter Rühren tropft man dann 52 ml Methylmagnesiumbromid in Diethylether (3 molare Lösung) zu, rührt weitere 30 Minuten und versetzt die graugefärbte Suspension mit einer Lösung aus 10,0 g (34,7 mmol) 11β-Fluorestra-4,6-dien-3,17-dion, 24,3 ml DMPU und 23 ml Trimethylsilylchlorid in 60 ml Dichlormethan. Nach vollendeter Zugabe läßt man noch 1,5 h zwischen -30 → -10 °C rühren, entfernt das Kältebad und gibt bei Raumtemperatur vorsichtig unter kräftigem Rühren 35 ml Essigester zu- Zur Aufarbeitung verdünnt man die Reaktionslösung mit Essigester, wäscht mit gesättigter wässriger Ammoniumchloridlösung kupferfrei und trocknet die organische Phase über Natriumsulfat. Das Rohprodukt wird an Kieselgel chromatografiert (Hexan-Essigester, Gradient bis 1:1), Ausbeute 3,1 g (30 %).

**11 β-Fluor-3-hydroxy-7α-methylestra-1,3,5(10)-trien-17-on**

Man versetzt 8,3 g (27,2 mmol) 11β-Fluor-7α-methylestr-4-en-3,17-dion in 260 ml Acetonitril mit 7,0 g (31,3 mmol) Kupfer(II)bromid und rührt das Reaktionsgemisch 7 Stunden bei Raumtemperatur. Zur Aufarbeitung verdünnt man die Reaktionslösung mit Essigester, wäscht die organische Phase mit wässriger Ammoniumchloridlösung, Natriumhydrogen-carbonatlösung, schließlich mit Wasser und trocknet über Natriumsulfat. Das Rohprodukt wird an Kieselgel chromatografiert (Toluol-Essigester, Gradient bis 7:3), Ausbeute 3,3 g (40 %), $[\alpha]_D$ +166,8° (c 0,5 Methanol).

**3-Acetyloxy-11β-fluor-7α-methylestra-1,3,5(10)-trien-17-on**

Man löst 3,0 g (9,9 mmol) 11β-Fluor-3-hydroxy-7α-methylestra-1,3,5(10)-trien-17-on in 12 ml Pyridin und 6 ml Essigsäureanhydrid auf und läßt über Nacht bei Raumtemperatur reagieren. Zur Aufarbeitung rührt man das Reaktionsgemisch in Eiswasser ein, saugt den Niederschlag ab, der anschließend in Essigester aufgenommen wird. Die organische Phase wird zunächst mit verdünnter Salzsäure, dann mit Wasser gewaschen und über Natriumsulfat getrocknet. Man erhält 3,4 g Produkt, das für die Weiterverarbeitung hinreichend rein ist.

**3-Acetyloxy-11β-fluor-7α-methylestra-1,3,5(10),16-tetraen**

Zu einer Lösung von 2,7 g (7,9 mmol) 3-Acetyloxy-11β-fluor-7α-methyletra-1,3,5(10)-trien-17-on in 40 ml getrocknetem Dichlormethan gibt man 3,8 g (18,4 mmol) 2,6-Di-tert.-butyl-4-methylpyridin, kühlt unter Schutzgasatmosphäre (Argon) auf 0 °C ab und tropft unter Rühren 2,64 ml (16 mmol) Trifluormethansulfonsäureanhydrid zu. Man entfernt das Kältebad und rührt noch 1,5 h bei Raumtemperatur nach. Zur Aufarbeitung verdünnt man mit Essigester, wäscht die organische Phase mit Wasser und trocknet über Natriumsulfat. Das Rohprodukt (6,0 g) wird sodann in 15 ml Dimethylformamid aufgenommen, bei Raumtemperatur mit 5,72 ml Tributylamin, 0,11 g (0,15 mmol) Bis(acetato)-bis(triphenylphosphin) palladium, 0,61 ml (16 mmol) Ameisensäure versetzt und 30 Minuten bei 60 °C Bad-temperatur gerührt (Argonatmosphäre). Zur Aufarbeitung gießt man in Eiswasser, extrahiert mit Essigester, wäscht die organische Phase zunächst mit verdünnter Salzsäure, dann mit Wasser und trocknet über Natriumsulfat. Das Rohprodukt wird an Kieselgel chromatografiert (Heptan-Aceton, Gradient bis 9:1), Ausbeute 1,71 g (66 %).

**11β-Fluor-7α-methylestra-1,3,5(10)-trien-3,16β-diol (33a)**

Eine Lösung von 1,67 g (5,22 mmol) 3-Acetyloxy-11β-fluor-7α-methylestra-1,3,5(10),16-tetraen in 30 ml DMSO und 2,4 ml Wasser ersetzt man unter Rühren bei 0 °C portionsweise mit 1,36 g N-Bromsuccinimid, entfernt nach vollständiger Zugabe von NBS das Kältebad und rührt noch 45 Minuten bei Raumtemperatur. Zur Aufarbeitung gießt man das Reaktionsgemisch in Eiswasser, extrahiert mit Essigester, wäscht die organische Phase mit Wasser und trocknet über Natriumsulfat. Das rohe Bromhydrin (2,3 g) wird dann in 25 ml trockenem Tetrahydrofuran aufgenommen, mit 5 ml (18,6 mmol) Tributylzinnhydrid, einer ersten Spatelspitze AIBN (insgesamt 200 mg verteilt über die Reaktionszeit) versetzt und 10 Stunden bei 80 °C Badtemperatur unter einer Argonatmosphäre gerührt. Zur Aufarbeitung verdünnt man mit Essigester, wäscht die organische Phase mit verdünnter Salzsäure, Wasser und trocknet über Natriumsulfat. Zur Verseifung löst man das Rohprodukt in 50 ml Methanol, 5 ml Dichlormethan und versetzt mit 2,0 g Kaliumcarbonat. Das Reaktionsgemisch wird 1,5 h unter Argon gerührt und zur Aufarbeitung in Eiswasser gegossen. Man stellt mit verdünnter Säure salzsauer und extrahiert mit Essigester. Die organische Phase wird mit Wasser gewaschen und über Natriumsulfat getrocknet. Das Rohprodukt wird an Kieselgel chromatografiert (Chloroform-tert. Butylmethylether, Gradient bis 95:5), Ausbeute 1,16 g (75 %), Schmelzpunkt 239-240 °C unter Zersetzung, $[\alpha]_D$ + 96,8° (c 0,51 Methanol).

**11β-Fluor-7α-methylestra-1,3,5(10)-trien-3,16α-diol (33b)**

Eine Lösung von 0,60 g (2,0 mmol) 11β-Fluor-7α-methylestra-1,3,5(10)-trien-3,16β-diol in 20 ml trockenem Tetrahydrofuran versetzt man mit 1,82 g (6,9 mmol) Triphenylphosphin und 0,26 ml Ameisensäure. Unter einer Argonatmosphäre tropft man zu dieser Lösung unter Rühren bei Raumtemperatur dann 1,10 ml DEAD. Nach einer Reaktionszeit von 30 Minuten versetzt man mit Wasser, extrahiert mit Essigester, wäscht die organische Phase mit Wasser und trocknet über Natriumsulfat. Das Rohprodukt wird chromatografisch an Kieselgel gereinigt (Dichlormethan-Essigester, Gradient bis 95:5). Die so erhaltenen Formiate werden in 15 ml Dichlormethan gelöst, mit 7,5 ml 3%iger methanolischer Kalilauge versetzt und 2 h bei Raumtemperatur unter Argon belassen. Zur Aufarbeitung versetzt man mit wässriger Essigsäure, extrahiert mit Essigester, wäscht die organische Phase mit Wasser und trocknet über Natriumsulfat. Das Rohprodukt wird durch Kristallisation aus Aceton/Hexan gereinigt. Ausbeute 0,45 g (75 %), Schmelzpunkt 221-222 °C unter Zersetzung, $[\alpha]_D$ +104,2° (c 0,52 Methanol).

In Analogie zu den Beispielen 3 und 4 wurden die nachfolgenden Verbindungen dargestellt:

7α-phenyl-estra- 1,3,5(10)-trien-3,16β-diol, Schmelzpunkt 239-241 °C (Aceton/Hexan);
7β-Phenyl-estra-1,3,5(10)-trien-3,16β-diol, Schmelzpunkt 171-173 °C (Aceton/Hexan);
7β-Phenyl-estra-1,3,5(10)-trien-3,16α-diol, amorph
7α-Ethyl-estra-1,3,5(10)-trien-3,16α-diol, Schmelzpunkt 192-193 °C (Aceton/Hexan);
7α-Ethyl-estra-1,3,5(10)-trien-3,16β-diol, amorph;
7β-Ethyl-estra-1,3,5(10)-trien-3,16α-diol, Schmelzpunkt 187-189 °C (Aceton/Hexan);
7β-Ethyl-estra-1,3,5(10)-trien-3,16β-diol, Schmelzpunkt 156-157 °C (Dichlormethan/Hexan).

Tabelle 1

| Estrogen | Struktur | hER α RBA* | hER β RBA* | ERß/ ERα | Rat uterus ER(RBA) | Rat prost. ER(RBA) | prost.ER/ uterusER |
|---|---|---|---|---|---|---|---|
| Estradiol | 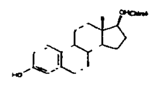 | 100 | 100 | 1 | 100 | 100 | 1 |
| Estron | 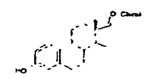 | 60 | 37 | 0.6 | 3 | 2 | 0.8 |
| 17α-Estradiol | 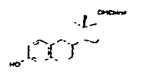 | 58 | 11 | 0.2 | 2.4 | 1.3 | 0.5 |
| Estriol | 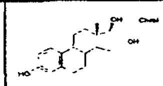 | 14 | 21 | 1.5 | 4 | 20 | 5 |
| 5-Androsten -diol | 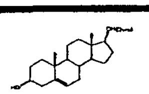 | 6 | 17 | 3 | 0.1 | 5 | 50 |
| Genistein | 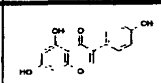 | 5 | 36 | 7 | 0.1 | 10 | 100 |
| Coumestrol | 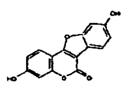 | 94 | 185 | 2 | 1.3 | 24 | 18 |

*: zitiert aus : Kuiper et al. (1996), Endocrinology 138: 863-870

Tabelle 2

| Verbindung bzw. Beispiel | Struktur | ER (RBA) Rattenuterus | ER (RBA) Rattenprostata | 50% Knochen-protektion bei [µg/Tier] | 50% Uterus-stimulation bei [µg/Tier] |
|---|---|---|---|---|---|
| 16α-Estradiol | 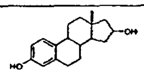 | 9 | 50 | 3 | 30 |
| 9 | 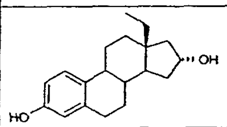 | 5.3 | 59 | | |
| 10 | 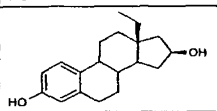 | 1 | 13 | | |
| 7β-Ethyl-estra-1,3,5(10)-trien-3,16β-diol | 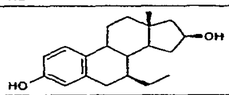 | 0.2 | 2 | | |
| 7α-Ethyl-estra-1,3,5(10)-trien-3,16β-diol | 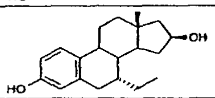 | 11 | 143 | | |
| 7β-Phenyl-estra-1,3,5(10)-trien-3,16β-diol | 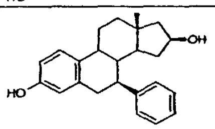 | 0.7 | 14 | | |
| 7α-Phenyl-estra-1,3,5(10)-trien-3,16β-diol | 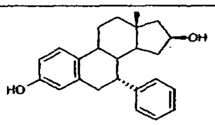 | 2.9 | 9 | | |
| 7β-Phenyl-estra-1,3,5(10)-trien-3,16α-diol | 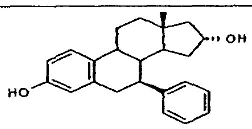 | 0.2 | 1.3 | | |
| 7β-Ethyl-estra-1,3,5(10)-trien-3,16α-diol | 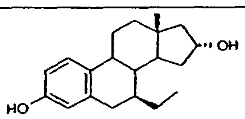 | 0.1 | 2.9 | | |
| 4 | 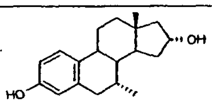 | 29 | 200 | | |
| 3 | 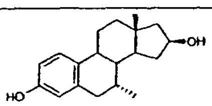 | 5.6 | 83 | | |

## Patentansprüche

**1.** 3,16-Dihydroxyestra-1,3,5(10)-trienderivate der allgemeinen Formel I

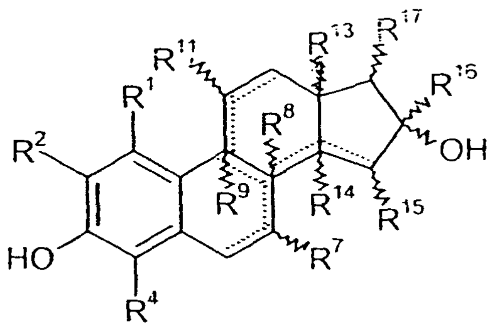

(I)

worin

| | |
|---|---|
| $R^7$ | ein α-oder β-ständiges Halogenatom, eine α-oder β-ständige, gerad- oder verzweigtkettige, gesättigte oder ungesättigte, gegebenenfalls teilweise oder vollständig fluorierte Alkylgruppe mit bis zu 10 Kohlenstoffatomen gegebenenfalls durch 1-5 Halogenatome, Hydroxygruppen oder $C_1$-$C_4$-Alkoxygruppen substituiert eine gerad- oder verzweigtkettige, gesättigte oder ungesättigte Alkoxygruppe mit bis zu 6 Kohlenstoffatomen oder einen gegebenenfalls substituierten Phenyl 1- oder 2-Naphthyl oder Heteroarylrest sowie |
| $R^{13}$ | ein α- oder β-ständige Methyl- oder Ethylgruppe |
| $R^1$, $R^2$, $R^4$, $R^8$, $R^9$, $R^{11}$, $R^{14}$, $R^{15}$, $R^{16}$ und $R^{17}$ | jeweils ein Wasserstoffatom |

bedeuten,
und wobei die Hydroxylgruppen an den C-Atomen 3 und 16 mit einer aliphatischen, gerad- oder verzweigtkettigen, gesättigten oder ungesättigten $C_1$-$C_{14}$-Mono- oder Polycarbonsäure oder einer aromatischen Carbonsäure oder mit einer α- oder β-Aminosäure verestert sein Konnen.

**2.** Verbindungen nach Anspruch 1, nämlich

7α-Fluor-estra-1,3,5(10)-trien-3,16α-diol,
7α-Methyl-estra-1,3,5(10)-trien-3,16α-diol
7α-Fluor-estra-1,3,5(10)-trien-3,16β-diol,
7α-Methyl-estra-1,3,5(10)-trien-3,16β-diol
7α-Ethyl-estra-1,3,5(10)-trien-3,16α-diol
7α-Propyl-estra-1,3,5(10)-trien-3,16α-diol
7α-*i*-Propyl-estra-1,3,5(10)-trien-3,16α-diol
7α-*i*-Propenyl-estra-1,3,5(10)-trien-3,16α-diol
7α-Phenyl-estra-1,3,5(10)-trien-3,16α-diol
7α-Methoxy-estra-1,3,5(10)-trien-3,16α-diol
7β-Ethyl-estra-1,3,5(10)-trien-3,16α-diol
7β-Propyl-estra-1,3,5(10)-trien-3,16α-diol
7β-*i*-Propyl-estra-1,3,5 (10)-trien-3,16α-diol
7β-*i*-Propenyl-estra-1,3,5(10)-trien-3,16α-diol
7β-Phenyl-estra-1,3,5(10)-trien-3,16α-diol
7β-Methoxy-estra-1,3,5(10)-trien-3,16α-diol
7α-Ethyl-estra-1,3,5(10)-trien-3,16β-diol
7α-Propyl-estra-1,3,5(10)-trien-3,16β-diol
7α-*i*-Propyl-estra-1,3,5(10)-trien-3,16β-diol
7α-*i*-Propenyl-estra-1,3,5(10)-trien-3,16β-diol
7α-Phenyl-estra-1,3,5(10)-trien-3,16β-diol

7α-Methoxy-estra-1,3,5(10)-trien-3,16β-diol
7β-Ethyl-estra-1,3,5(10)-trien-3,16β-diol
7β-Propyl-estra-1,3,5(10)-trien-3,16β-diol
7β-*i*-Propyl-estra-1,3,5(10)-trien-3,16β-diol
7β-*i*-Propenyl-estra-1,3,5(10)-trien-3,16β-diol
7β-Phenyl-estra-1,3,5(10)-trien-3,16β-diol
7β-Methoxy-estra-1,3,5(10)-trien-3,16β-diol
7α-Phenyl-8α-estra-1,3,5(10)-trien-3,16α-diol
18a-Homo-7α-phenyl-estra-1,3,5(10)-trien-3,16α-diol,
7α-Phenyl-8α-estra-1,3,5(10)-trien-3,16β-diol
18a-Homo-7α-phenyl-estra-1,3,5(10)-trien-3,16β-diol,
7α-Methyl-18a-homoestra-1,3,5(10)-trien-3,16α-diol
7α-Methyl-18a-homoestra-1,3,5(10)-trien-3,16β-diol
7α-Ethyl-18a-homoestra-1,3,5(10)-trien-3,16α-diol
7α-Ethyl-18a-homoestra-1,3,5(10)-trien-3,16β-diol

**3.** Verbindungen nach Anspruch 2, nämlich

7α-Fluor-estra-1,3,5(10)-trien-3,16α-diol
7α-Methyl-estra-1,3,5(10)-trien-3,16β-diol
7α-Methyl-estra-1,3,5(10)-trien-3,16α-diol
7α-Phenyl-estra-1,3,5(10)-trien-3,16β-diol
7β-Phenyl-estra- 1,3,5(10)-trien-3,16β-diol
7β-Phenyl-estra-1,3,5(10)-trien-3,16α-diol
7α-Ethyl-estra-1,3,5 (10)-trien-3,16β-diol
7β-Ethyl-estra-1,3,5 (10)-trien-3,16α-diol
7β-Ethyl-estra-1,3,5(10)-trien-3,16β-diol

**4.** Verwendung der 3,16-Dihydroxyestra-1,3,5(10)-trienderivate der allgemeinen Formel I gemäß Anspruch 1 als ERβ selektive Estrogene zur Herstellung eines Arzneimittels zur Behandlung estrogendefizienz-bedingter Krankheiten und Zustände bei der Frau und beim Mann.

**5.** Verwendung nach Anspruch 4, zur Behandlung von peri- und postmenopausalen Beschwerden.

**6.** Verwendung nach Anspruch 4, zur Behandlung von peri- und postandropausalen Beschwerden.

**7.** Verwendung nach Anspruch 5, zur Vorbeugung gegen und Behandlung von Hitzewallungen, Schlafstörungen, Reizbarkeit, Stimmungsschwankungen, Inkontinenz, Vaginalatrophie und hormondefizienzbedingter Gemütserkrankungen.

**8.** Verwendung nach Anspruch 4, zur Vorbeugung und Behandlung von Erkrankungen im Urogenitaltrakt.

**9.** Verwendung nach Anspruch 4, zur Vorbeugung und Therapie von Magen- und Darmerkrankungen.

**10.** Verwendung nach Anspruch 9, zur Vorbeugung und Therapie von Ulcera und hämorrhagischen Diathesen im Magendarmtrakt.

**11.** Verwendung nach Anspruch 4, zur Vorbeugung und Therapie von Neoplasien.

**12.** Verwendung nach Anspruch 4, für die in-vitro Behandlung der männlichen Infertilität.

**13.** Verwendung nach Anspruch 4, für die in-vivo Behandlung der männlichen Infertilität.

**14.** Verwendung nach Anspruch 4, für die in-vitro Behandlung der weiblichen Infertilität.

**15.** Verwendung nach Anspruch 4, für die in-vivo Behandlung der weiblichen Infertilität.

**16.** Verwendung nach Anspruch 4, für die Hormonersatz-Therapie (HRT).

**17.** Verwendung nach Anspruch 4, für die Therapie von hormondefizienz bedingten Beschwerden bei chirurgisch, medikamentös oder anders bedingter ovarieller Dysfunktion.

**18.** Verwendung nach Anspruch 4, zur Prophylaxe und Therapie eines hormondefizienzbedingten Knochenmasseverlustes.

**19.** Verwendung nach Anspruch 18, zur Prophylaxe und Therapie der Osteoporose.

**20.** Verwendung nach Anspruch 4, zur Vorbeugung gegen und Therapie von Herzkreislauferkrankungen.

**21.** Verwendung nach Anspruch 4, zur Vorbeugung gegen und Behandlung von Gefäßerkrankungen.

**22.** Verwendung nach Anspruch 21, zur Vorbeugung gegen und Behandlung von Atherosklerose.

**23.** Verwendung nach Anspruch 21, zur Vorbeugung und Behandlung neointimaler Hyperplasien.

**24.** Verwendung nach Anspruch 4, zur Vorbeugung und Behandlung hormondefizienzbedingter neurodegenerativer Erkrankungen.

**25.** Verwendung nach Anspruch 4, zur Vorbeugung und Behandlung der Alzheimerschen Krankheit sowie hormondefizienzbedingter Beeinträchtigung von Gedächtnis- und Lernfähigkeit.

**26.** Verwendung nach Anspruch 4, zur Behandlung von entzündlichen Erkrankungen und Erkrankungen des Immunsystems.

**27.** Verwendung nach Anspruch 4, zur Vorbeugung und Behandlung der benignen Prostatahyperplasie (BPH).

**28.** Phärmazeutische Zusammensetzungen, enthaltend mindestens eine Verbindung gemäß einem der Ansprüche 1 bis 3, sowie einen pharmazeutisch verträglichen Träger.

## Claims

**1.** 3,16-Dihydroxyestra-1,3,5(10)-triene derivatives of the general formula I

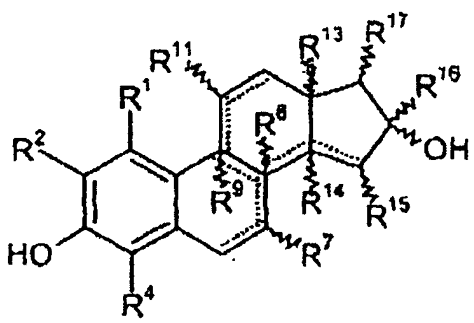

(I)

where

| | |
|---|---|
| $R^7$ | is an α- or β-oriented halogen atom, an α- or β-oriented, straight- or branched-chain, saturated or unsaturated, optionally partly or completely fluorinated alkyl group having up to 10 carbon atoms, optionally substituted by 1 - 5 halogen atoms, hydroxy groups or $C_1$-$C_4$-alkoxy groups, a straight- or branched-chain, saturated or unsaturated alkoxy group having up to 6 carbon atoms or an optionally substituted phenyl, 1- or 2-naphthyl or heteroaryl rad- |

ical and

| | |
|---|---|
| $R^{13}$ | is an α- or β-oriented methyl or ethyl group |
| $R^1$, $R^2$, $R^4$, $R^8$, $R^9$, $R^{11}$, $R^{14}$, $R^{15}$, $R^{16}$ and $R^{17}$ | are each a hydrogen atom, |

and where the hydroxyl groups on carbon atoms 3 and 16 may be esterified with an aliphatic, straight- or branched-chain, saturated or unsaturated $C_1$-$C_{14}$-mono- or polycarboxylic acid or an aromatic carboxylic acid or with an α- or β-amino acid.

**2.** Compounds according to Claim 1, namely

7α-fluoroestra-1,3,5(10)-triene-3,16α-diol
7α-methylestra-1,3,5(10)-triene-3,16α-diol
7α-fluoroestra-1,3,5(10)-triene-3,16β-diol
7α-methylestra-1,3,5(10)-triene-3,16β-diol
7α-ethylestra-1,3,5(10)-triene-3,16α-diol
7α-propylestra-1,3,5(10)-triene-3,16α-diol
7α-*i*-propylestra-1,3,5(10)-triene-3,16α-diol
7α-*i*-propenylestra-1,3,5(10)-triene-3,16α-diol
7α-phenylestra-1,3,5(10)-triene-3,16α-diol
7α-methoxyestra-1,3,5(10)-triene-3,16α-diol
7β-ethylestra-1,3,5(10)-triene-3,16α-diol
7β-propylestra-1,3,5(10)-triene-3,16α-diol
7β-*i*-propylestra-1,3,5(10)-triene-3,16α-diol
7β-*i*-propenylestra-1,3,5(10)-triene-3,16α-diol
7β-phenylestra-1,3,5(10)-triene-3,16α-diol
7β-methoxyestra-1,3,5(10)-triene-3,16α-diol
7α-ethylestra-1,3,5(10)-triene-3,16β-diol
7α-propylestra-1,3,5(10)-triene-3,16β-diol
7α-*i*-propylestra-1,3,5(10)-triene-3,16β-diol
7α-*i*-propenylestra-1,3,5(10)-triene-3,16β-diol
7α-phenylestra-1,3,5(10)-triene-3,16β-diol
7α-methoxyestra-1,3,5(10)-triene-3,16β-diol
7β-ethylestra-1,3,5(10)-triene-3,16β-diol
7β-propylestra-1,3,5(10)-triene-3,16β-diol
7β-*i*-propylestra-1,3,5(10)-triene-3,16β-diol
7β-*i*-propenylestra-1,3,5 (10)-triene-3,16β-diol
7β-phenylestra-1,3,5(10)-triene-3,16β-diol
7β-methoxyestra-1,3,5(10)-triene-3,16β-diol
7α-phenyl-8α-estra-1,3,5(10)-triene-3,16α-diol
18a-homo-7α-phenylestra-1,3,5(10)-triene-3,16α-diol
7α-phenyl-8α-estra-1,3,5(10)-triene-3,16β-diol
18a-homo-7α-phenylestra-1,3,5(10)-triene-3,16β-diol
7α-methyl-18a-homoestra-1,3,5(10)-triene-3,16α-diol
7α-methyl-18a-homoestra-1,3,5(10)-triene-3,16β-diol
7α-ethyl-18a-homoestra-1,3,5(10)-triene-3,16α-diol
7α-ethyl-18a-homoestra-1,3,5(10)-triene-3,16β-diol

**3.** Compounds according to Claim 2, namely

7α-fluoroestra-1,3,5(10)-triene-3,16α-diol
7α-methylestra-1,3,5(10)-triene-3,16β-diol
7α-methylestra-1,3,5(10)-triene-3,16α-diol
7α-phenylestra-1,3,5(10)-triene-3,16β-diol
7β-phenylestra-1,3,5(10)-triene-3,16β-diol
7β-phenylestra-1,3,5(10)-triene-3,16α-diol
7α-ethylestra-1,3,5(10)-triene-3,16β-diol

7β-ethylestra-1,3,5(10)-triene-3,16α-diol
7β-ethylestra-1, 3, 5 (10) -triene-3, 16β-diol.

**4.** Use of the 3,16-dihydroxyestra-1,3,5(10)-triene derivatives of the general formula I according to Claim 1 as ER-β selective estrogens for producing a medicament for the treatment of diseases and conditions caused by estrogen deficiency in women and men.

**5.** Use according to Claim 4 for the treatment of peri- and postmenopausal symptoms.

**6.** Use according to Claim 4 for the treatment of peri- and postandropausal symptoms.

**7.** Use according to Claim 5 for the prevention and treatment of hot flushes, sleep disorders, irritability, mood fluctuations, incontinence, vaginal atrophy and affective disorders caused by hormone deficiency.

**8.** Use according to Claim 4 for the prevention and treatment of disorders in the urogenital tract.

**9.** Use according to Claim 4 for the prevention and therapy of gastrointestinal disorders.

**10.** Use according to Claim 9 for the prevention and therapy of ulcers and haemorrhagic diatheses in the gastrointestinal tract.

**11.** Use according to Claim 4 for the prevention and therapy of neoplasias.

**12.** Use according to Claim 4 for the in vitro treatment of male infertility.

**13.** Use according to Claim 4 for the in vivo treatment of male infertility.

**14.** Use according to Claim 4 for the in vitro treatment of female infertility.

**15.** Use according to Claim 4 for the in vivo treatment of female infertility.

**16.** Use according to Claim 4 for hormone replacement therapy (HRT).

**17.** Use according to Claim 4 for the therapy of symptoms caused by hormone deficiency associated with ovarian dysfunction related to surgery, medicines or other factors.

**18.** Use according to Claim 4 for the prophylaxis and therapy of a loss of bone mass caused by hormone deficiency.

**19.** Use according to Claim 18 for the prophylaxis and therapy of osteoporosis.

**20.** Use according to Claim 4 for the prevention and therapy of cardiovascular disorders.

**21.** Use according to Claim 4 for the prevention and treatment of vascular disorders.

**22.** Use according to Claim 21 for the prevention and treatment of atherosclerosis.

**23.** Use according to Claim 21 for the prevention and treatment of neointimal hyperplasias.

**24.** Use according to Claim 4 for the prevention and treatment of neurodegenerative disorders caused by hormone deficiency.

**25.** Use according to Claim 4 for the prevention and treatment of Alzheimer's disease and memory and learning impairment caused by hormone deficiency.

**26.** Use according to Claim 4 for the treatment of inflammatory disorders and disorders of the immune system.

**27.** Use according to Claim 4 for the prevention and treatment of benign prostate hyperplasia (BPH).

**28.** Pharmaceutical compositions comprising at least one compound according to any of Claims 1 to 3 and a pharmaceutically acceptable carrier.

## Revendications

**1.** Dérivés de 3,16-dihydroxyestra-1,3,5(10)-triène de formule générale I

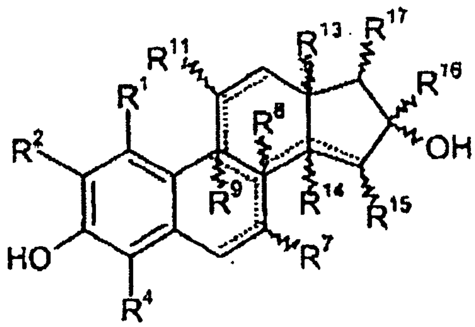

(I)

dans laquelle

| | |
|---|---|
| $R^7$ | représente un atome d'halogène en position α ou β, un groupe alkyle en position α ou β, à chaîne droite ou ramifiée, saturé ou insaturé, éventuellement partiellement ou totalement fluoré, ayant jusqu'à 10 atomes de carbone, éventuellement substitué par 1-5 atomes d'halogène, groupes hydroxy ou groupes alcoxy en $C_1$-$C_4$, un groupe alcoxy à chaîne droite ou ramifiée, saturé ou insaturé, ayant jusqu'à 6 atomes de carbone, ou un radical phényle, 1- ou 2-naphtyle ou hétéroaryle éventuellement substitué, et |
| $R^{13}$ | représente un groupe méthyle ou éthyle en position α ou β, |
| $R^1$, $R^2$, $R^4$, $R^8$, $R^9$, $R^{11}$, $R^{14}$, $R^{15}$, $R^{16}$ et $R^{17}$ | représentent chacun un atome d'hydrogène, |

et dans laquelle les groupes hydroxy sur les atomes de carbone 3 et 16 peuvent être estérifiés par un acide mono- ou polycarboxylique en $C_1$-$C_{14}$ aliphatique, à chaîne droite ou ramifiée, saturé ou insaturé, ou un acide carboxylique aromatique ou par un acide α- ou β-aminé.

**2.** Composés selon la revendication 1, à savoir

7α-fluoro-estra-1,3,5(10)-triène-3,16α-diol,
7α-méthyl-estra-1,3,5(10)-triène-3,16α-diol,
7α-fluoro-estra-1,3,5(10)-triène-3,16β-diol,
7α-méthyl-estra-1,3,5(10)-triène-3,16β-diol,
7α-éthyl-estra-1,3,5(10)-triène-3,16α-diol,
7α-propyl-estra-1,3,5(10)-triène-3,16α-diol,
7α-isopropyl-estra-1,3,5(10)-triène-3,16α-diol,
7α-isopropényl-estra-1,3,5(10)-triène-3,16α-diol,
7α-phényl-estra-1,3,5(10)-triène-3,16α-diol,
7α-méthoxy-estra-1,3,5(10)-triène-3,16α-diol,
7β-éthyl-estra-1,3,5(10)-triène-3,16α-diol,
7β-propyl-estra-1,3,5(10)-triène-3,16α-diol,
7β-isopropyl-estra-1,3,5(10)-triène-3,16α-diol,
7β-isopropényl-estra-1,3,5(10)-triène-3,16α-diol,
7β-phényl-estra-1,3,5(10)-triène-3,16α-diol,
7β-méthoxy-estra-1,3,5(10)-triène-3,16α-diol,
7α-éthyl-estra-1,3,5(10)-triène-3,16β-diol,

7α-propyl-estra-1,3,5(10)-triène-3,16β-diol,
7α-isopropyl-estra-1,3,5(10)-triène-3,16β-diol,
7α-isopropényl-estra-1,3,5(10)-triène-3,16β-diol,
7α-phényl-estra-1,3,5(10)-triène-3,16β-diol,
7α-méthoxy-estra-1,3,5(10)-triène-3,16β-diol,
7β-éthyl-estra-1,3,5(10)-triène-3,16β-diol,
7β-propyl-estra-1,3,5(10)-triène-3,16β-diol,
7β-isopropyl-estra-1,3,5(10)-triène-3,16β-diol,
7β-isopropényl-estra-1,3,5(10)-triène-3,16β-diol,
7β-phényl-estra-1,3,5(10)-triène-3,16β-diol,
7β-méthoxy-estra-1,3,5(10)-triène-3,16β-diol,
7α-phényl-8α-estra-1,3,5(10)-triène-3,16α-diol,
18a-homo-7α-phényl-estra-1,3,5(10)-triène-3,16α-diol,
7α-phényl-8α-estra-1,3,5(10)-triène-3,16β-diol,
18a-homo-7α-phényl-estra-1,3,5(10)-triène-3,16β-diol,
7α-méthyl-18a-homoestra-1,3,5(10)-triène-3,16α-diol,
7α-méthyl-18a-homoestra-1,3,5(10)-triène-3,16β-diol,
7α-éthyl-18a-homoestra-1,3,5(10)-triène-3,16α-diol,
7α-éthyl-18a-homoestra-1,3,5(10)-triène-3,16β-diol.

3. Composé selon la revendication 2, à savoir

7α-fluoro-estra-1,3,5(10)-triène-3,16α-diol,
7α-méthyl-estra-1,3,5(10)-triène-3,16β-diol,
7α-méthyl-estra-1,3,5(10)-triène-3,16α-diol,
7α-phényl-estra-1,3,5(10)-triène-3,16β-diol,
7β-phényl-estra-1,3,5(10)-triène-3,16β-diol,
7β-phényl-estra-1,3,5(10)-triène-3,16α-diol,
7α-éthyl-estra-1,3,5(10)-triène-3,16β-diol,
7β-éthyl-estra-1,3,5(10)-triène-3,16α-diol,
7β-éthyl-estra-1,3,5(10)-triène-3,16β-diol.

4. Utilisation des dérivés de 3,16-dihydroxy-estra-1,3,5(10)-triène de formule générale I selon la revendication 1, en tant qu' oestrogène ER-β-sélectif pour la fabrication d'un médicament destiné au traitement de maladies et d'états dus à une déficience en oestrogènes chez la femme et chez l'homme.

5. Utilisation selon la revendication 4, pour le traitement de troubles péri- et post-ménopausiques.

6. Utilisation selon la revendication 4, pour le traitement de troubles péri- et post-andropausiques.

7. Utilisation selon la revendication 5, pour la prévention et le traitement de bouffées de chaleur, de troubles du sommeil, de l'irritabilité, de sautes d'humeur, de l'incontinence, de l'atrophie vaginale et de maladies mentales dues à une déficience hormonale.

8. Utilisation selon la revendication 4, pour la prévention et le traitement de maladies du tractus uro-génital.

9. Utilisation selon la revendication 4, pour la prévention et la thérapie de maladies gastriques et intestinales.

10. Utilisation selon la revendication 9, pour la prévention et la thérapie d'ulcères et de diathèses hémorragiques dans le tractus gastro-intestinal.

11. Utilisation selon la revendication 4, pour la prévention et la thérapie de néoplasies.

12. Utilisation selon la revendication 4, pour le traitement in vitro de l'infertilité masculine.

13. Utilisation selon la revendication 4, pour le traitement in vivo de l'infertilité masculine.

14. Utilisation selon la revendication 4, pour le traitement in vitro de l'infertilité féminine.

**15.** Utilisation selon la revendication 4, pour le traitement in vivo de l'infertilité féminine.

**16.** Utilisation selon la revendication 4, pour la thérapie hormonale substitutive (HRT).

**17.** Utilisation selon la revendication 4, pour la thérapie de troubles dues à une déficience hormonale dans un dysfonctionnement ovarien d'origine chirurgicale, médicamenteuse ou autre.

**18.** Utilisation selon la revendication 4, pour la prophylaxie et la thérapie d'une perte de masse osseuse due à une déficience hormonale.

**19.** Utilisation selon la revendication 18, pour la prophylaxie et la thérapie de l'ostéoporose.

**20.** Utilisation selon la revendication 4, pour la prévention et la thérapie de maladies cardiovasculaires.

**21.** Utilisation selon la revendication 4, pour la prévention et le traitement de maladies vasculaires.

**22.** Utilisation selon la revendication 21, pour la prévention et le traitement de l'athérosclérose.

**23.** Utilisation selon la revendication 21, pour la prévention et le traitement d'hyperplasies néo-intimales.

**24.** Utilisation selon la revendication 4, pour la prévention et le traitement de maladies neurodégénératives dues à une déficience hormonale.

**25.** Utilisation selon la revendication 4, pour la prévention et le traitement de la maladie d'Alzheimer ainsi que d'altération de la mémoire et de l'apprentissage, due à une déficience hormonale.

**26.** Utilisation selon la revendication 4, pour le traitement de maladies inflammatoires et de maladies du système immunitaire.

**27.** Utilisation selon la revendication 4, pour la prévention et le traitement de l'hyperplasie bénigne de la prostate (BPH).

**28.** Compositions pharmaceutiques contenant au moins un composé selon l'une quelconque des revendications 1 à 3, ainsi qu'un véhicule pharmaceutiquement acceptable.